(19) Europäisches Patentamt / European Patent Office / Office européen des brevets

(11) **EP 4 585 190 A1**

(12) **EUROPEAN PATENT APPLICATION**
published in accordance with Art. 153(4) EPC

(43) Date of publication:
**16.07.2025 Bulletin 2025/29**

(21) Application number: **23863225.1**

(22) Date of filing: **06.09.2023**

(51) International Patent Classification (IPC):
**A61F 2/64** $^{(2006.01)}$     **A61F 2/70** $^{(2006.01)}$

(52) Cooperative Patent Classification (CPC):
**A61F 2/64; A61F 2/70**

(86) International application number:
**PCT/JP2023/032583**

(87) International publication number:
**WO 2024/053687 (14.03.2024 Gazette 2024/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **07.09.2022   JP 2022142463**

(71) Applicant: **HONDA MOTOR CO., LTD.**
**Minato-ku**
**Tokyo 107-8556 (JP)**

(72) Inventors:
• **KOTANI Yoshiaki**
  **Wako-shi, Saitama 351-0193 (JP)**
• **ONO Hiromi**
  **Wako-shi, Saitama 351-0193 (JP)**
• **MATSUMOTO Satoki**
  **Wako-shi, Saitama 351-0193 (JP)**
• **OTA Hiroshi**
  **Kawasaki-shi, Kanagawa 212-0013 (JP)**
• **OKANO Masahiro**
  **Tokyo 140-0004 (JP)**

(74) Representative: **Weickmann & Weickmann
PartmbB
Postfach 860 820
81635 München (DE)**

(54) **COUPLING DEVICE, COUPLING DEVICE CONTROL METHOD, COUPLING DEVICE CONTROL PROGRAM, AND RECORDING MEDIUM HAVING SAID CONTROL PROGRAM STORED THEREON**

(57)     A control unit (10) of an electric prosthetic leg (1) controls a motor (M) based on a torque target value which is a target value of a torque related to a torque of an enlarging and reducing device (200), during a stance phase which is a weighted state, and controls the motor (M) based on a position target value which is a target value of a position of the enlarging and reducing device (200), during a swing phase which is a non-weighted state.

FIG. 20

EP 4 585 190 A1

**Description**

TECHNICAL FIELD

The present invention relates to a joint device, a knee joint device, a joint device control method, a joint device control program, and a storage medium storing the control program.

BACKGROUND ART

In the related art, as an example of a knee joint device, a prosthetic leg device that is attached to a leg of a robot or a person who has cut his/her leg due to an accident or a disease. For example, Patent Literature 1 discloses an electric prosthetic leg equipped with a transmission having two power transmission paths having different transmission ratios in order to smoothly ascend and descend stairs. It is disclosed that in the electric prosthetic leg, the power transmission path varies when a knee joint mechanism is stretched in a state where a load is applied to the electric prosthetic leg and when the knee joint mechanism is stretched in a state where no load is applied.

CITATION LIST

PATENT LITERATURE

Patent Literature 1: WO2021/251500A

SUMMARY OF INVENTION

TECHNICAL PROBLEM

In such a joint device, it is required to consider on how to perform control in a specific use state such as a weighted state where a load is applied and a non-weighted state where no load is applied.

The present invention is to provide a joint device, a joint device control method, a joint device control program, and a storage medium storing the control program, capable of performing appropriate control in either a weighted state or a non-weighted state.

SOLUTION TO PROBLEM

The present invention provides a joint device including:

a first member;
a second member;
a coupling portion configured to couple the first member and the second member such that a formed angle formed between the first member and the second member is variable; and
an enlarging and reducing device capable of enlarging and reducing the formed angle formed between the first member and the second member, in which
the enlarging and reducing device includes a power source, a power transmission unit configured to transmit power of the power source, and a control unit configured to control the power source,
the joint device is provided to transition between a weighted state where a weight applied from an outside is received and a non-weighted state where no weight is received, and
the control unit,

(A) when the joint device is in the weighted state,
controls the power source based on a torque target value which is a target value of a torque related to a torque for the enlarging and reducing device to enlarge or reduce the formed angle, and
(B) when the joint device is in the non-weighted state,
controls the power source based on a position target value which is a target value of a position related to the formed angle enlarged or reduced by the enlarging and reducing device.

Further, the present invention provides a joint device including:

a first member;

a second member;

a coupling portion configured to couple the first member and the second member such that a formed angle formed between the first member and the second member is variable; and

an enlarging and reducing device capable of enlarging and reducing the formed angle formed between the first member and the second member, in which

the enlarging and reducing device includes a power source, a power transmission unit configured to transmit power of the power source, a connection and disconnection mechanism disposed on a power transmission path of the power transmission unit and configured to switch between connection and disconnection of power in the power transmission path, and a control unit configured to control the power source and the connection and disconnection mechanism,

the joint device is provided to transition between a weighted state where a weight applied from an outside is received and a non-weighted state where no weight is received, and

the control unit,

    (A) when the joint device is in the weighted state,

        controls the connection and disconnection mechanism to a connection state where the power transmission path is connected, and

        controls the power source such that the power source does not generate power or is stopped, and

    (B) when the joint device is in the non-weighted state,

        controls the connection and disconnection mechanism to a disconnection state where the power transmission path is disconnected, and

        controls the power source such that the power source does not generate power or is stopped.

The present invention provides a joint device control method for controlling a joint device including:

a first member;

a second member;

a coupling portion configured to couple the first member and the second member such that a formed angle formed between the first member and the second member is variable; and

an enlarging and reducing device capable of enlarging and reducing the formed angle formed between the first member and the second member, in which

the enlarging and reducing device includes a power source, and a power transmission unit configured to transmit power of the power source,

the joint device is provided to transition between a weighted state where a weight applied from an outside is received and a non-weighted state where no weight is received, and

the control method includes the steps of:

    (A) when the joint device is in the weighted state,
    controlling the power source based on a torque target value which is a target value of a torque related to a torque for the enlarging and reducing device to enlarge or reduce the formed angle; and
    (B) when the joint device is in the non-weighted state,
    controlling the power source based on a position target value which is a target value of a position related to the formed angle enlarged or reduced by the enlarging and reducing device.

Further, the present invention provides a joint device control program for controlling a joint device including:

a first member;

a second member;

a coupling portion configured to couple the first member and the second member such that a formed angle formed between the first member and the second member is variable; and

an enlarging and reducing device capable of enlarging and reducing the formed angle formed between the first member and the second member, in which

the enlarging and reducing device includes a power source, and a power transmission unit configured to transmit power of the power source,

the joint device is provided to transition between a weighted state where a weight applied from an outside is received and a non-weighted state where no weight is received, and

the control program causes a computer to perform the steps of:

(A) when the joint device is in the weighted state,
controlling the power source based on a torque target value which is a target value of a torque related to a torque for the enlarging and reducing device to enlarge or reduce the formed angle; and
(B) when the joint device is in the non-weighted state,
controlling the power source based on a position target value which is a target value of a position related to the formed angle enlarged or reduced by the enlarging and reducing device.

Further, the present invention provides a computer-readable storage medium storing the above-described control program.

ADVANTAGEOUS EFFECTS OF INVENTION

According to the present invention, the control can be appropriately performed in both the weighted state and the non-weighted state.

BRIEF DESCRIPTION OF DRAWINGS

[FIG. 1] FIG. 1 is a perspective view of an electric prosthetic leg 1 according to a first embodiment as viewed obliquely from the front.
[FIG. 2] FIG. 2 is an exploded perspective view of the electric prosthetic leg 1.
[FIG. 3] FIG. 3 is a cross-sectional view of the electric prosthetic leg 1.
[FIG. 4] FIG. 4 is a cross-sectional view of an expansion-contraction device 140.
[FIG. 5] FIG. 5 is a cross-sectional view of a main part showing a bent state of the electric prosthetic leg 1.
[FIG. 6] FIG. 6 is a cross-sectional view of a main part showing a maximum bent state of the electric prosthetic leg 1.
[FIG. 7] FIG. 7 is a cross-sectional view of a two-way clutch.
[FIG. 8] FIG. 8 is a perspective view of a retainer 282.
[FIG. 9] FIG. 9 is a view showing operations of an operation mechanism 240, in which (A) of FIG. 9 shows a state where a connection and disconnection unit 212 and a connection and disconnection unit 222 are in an off state, and (B) of FIG. 9 shows a state where the connection and disconnection unit 212 is in the off state and the connection and disconnection unit 222 is in an on state, and (C) of FIG. 9 shows a state where the connection and disconnection unit 212 is in the on state and the connection and disconnection unit 222 is in the off state.
[FIG. 10] (A) of FIG. 10 is a cross-sectional view showing a state where the connection and disconnection unit 222 is in the off state, and (B) of FIG. 10 is a view showing a position of an operation rod 241 at that time.
[FIG. 11] (A) of FIG. 11 is a cross-sectional view showing a state where the connection and disconnection unit 222 is operated from the off state to the on state, and (B) of FIG. 11 is a view showing a position of the operation rod 241 at that time.
[FIG. 12] (A) of FIG. 12 is a cross-sectional view showing a normal-rotation on state of the connection and disconnection unit 222, and (B) of FIG. 12 is a view showing a position of the operation rod 241 at that time.
[FIG. 13] (A) of FIG. 13 is a cross-sectional view showing a reverse-rotation on state of the connection and disconnection unit 222, and (B) of FIG. 13 is a view showing a position of the operation rod 241 at that time.
[FIG. 14] (A) of FIG. 14 is a cross-sectional view showing a state where the connection and disconnection unit 222 is operated from the on state to the off state, and (B) of FIG. 14 is a view showing a position of the operation rod 241 at that time.
[FIG. 15] FIG. 15 is a cross-sectional view of the electric prosthetic leg 1 of a second embodiment.
[FIG. 16] FIG. 16 is a functional block diagram of the electric prosthetic leg 1.
[FIG. 17] FIG. 17 is a diagram showing actions (stair ascending actions) of a human and the electric prosthetic leg when ascending stairs.
[FIG. 18] FIG. 18 is a diagram showing actions (flat ground walking actions) of the human and the electric prosthetic leg when walking on a flat ground.
[FIG. 19] (A) of FIG. 19 is a diagram showing a knee angle, (B) of FIG. 19 is a diagram showing a thigh angle, and (C) of FIG. 19 is a diagram showing a lower leg angle.
[FIG. 20] FIG. 20 is a table summarizing phases and corresponding control methods in a stair ascending mode and a flat ground and stair descending mode.
[FIG. 21] FIG. 21 is a diagram showing torque control.

[FIG. 22] FIG. 22 is a diagram showing an initial swing phase_method 1.

[FIG. 23] FIG. 23 is a diagram showing an initial swing phase_method 2.

[FIG. 24] FIG. 24 is a diagram showing a terminal swing phase.

[FIG. 25] FIG. 25 is a diagram showing a transition between repeated actions of an initial swing phase, the terminal swing phase, and a stance phase.

[FIG. 26] FIG. 26 is a diagram showing an example of an electric circuit 16 connecting a battery B and a motor M.

[FIG. 27] FIG. 27 is a table showing states of a switch SW1, transistors Tr1 to Tr3, and the motor M in each walking mode.

[FIG. 28] FIG. 28 is a table showing on/off states of the transistors Tr1 to Tr3 according to a body weight of a user.

DESCRIPTION OF EMBODIMENTS

Hereinafter, an electric prosthetic leg as an embodiment of a joint device of the present invention will be described below with reference to the drawings. In the following description, a front-rear direction, a left-right direction, and an upper-lower direction are defined with reference to a user of the electric prosthetic leg. In the drawings, a front side of the electric prosthetic leg is denoted by Fr, a rear side is denoted by Rr, a left side is denoted by L, a right side is denoted by R, an upper side is denoted by U, and a lower side is denoted by D.

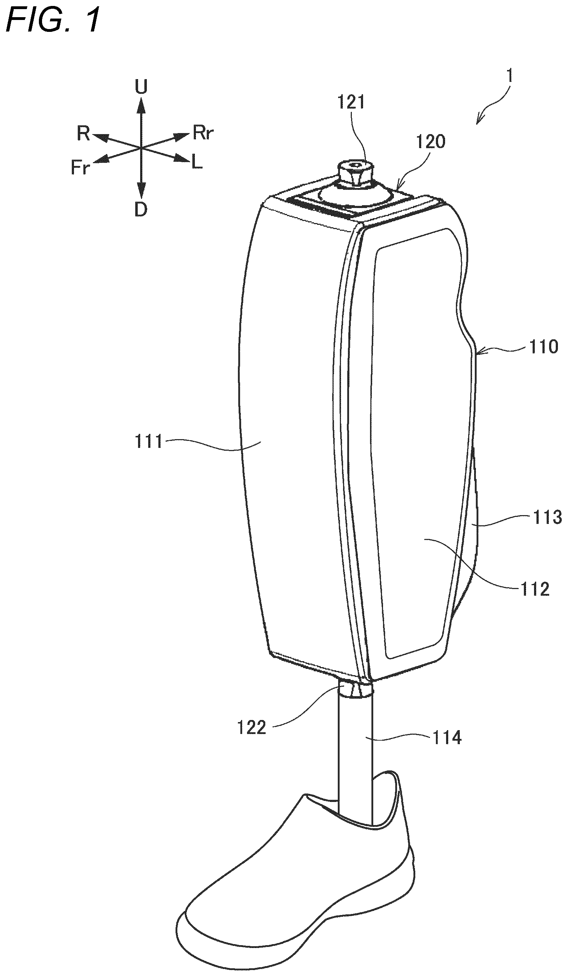

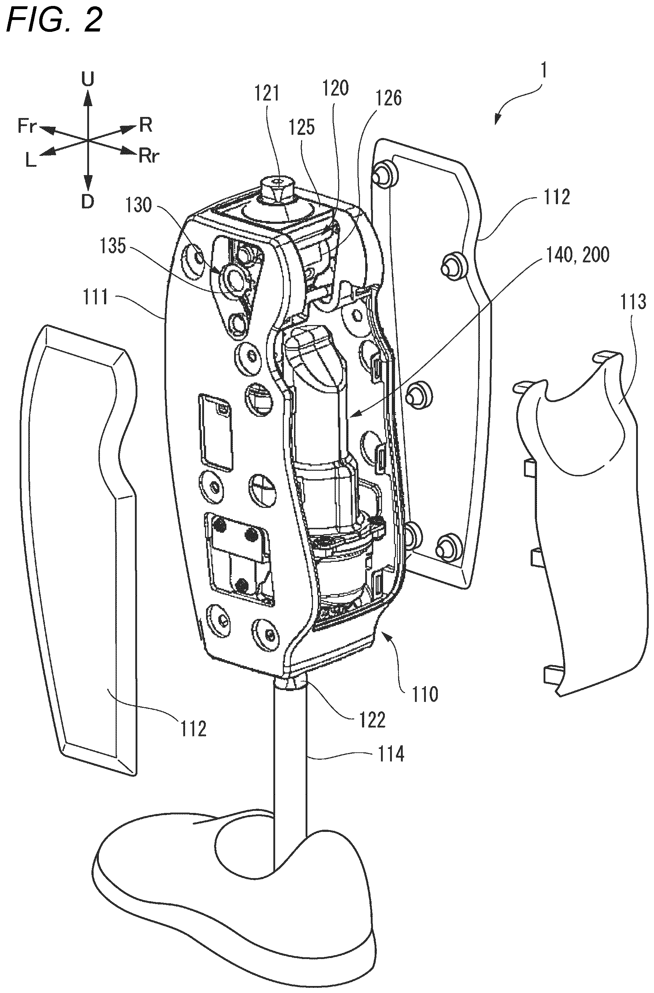

As shown in FIGs. 1 to 4, an electric prosthetic leg 1 according to the present embodiment is a prosthetic leg that is attached to a leg of a person who does not have a knee. The electric prosthetic leg 1 includes: a below-knee member 110 positioned on a lower side of the knee, an above-knee member 120 positioned on an upper side of the knee and attached to a thigh, a knee joint mechanism 130 that couples the below-knee member 110 and the above-knee member 120 such that a formed angle formed between the below-knee member 110 and the above-knee member 120 is variable, an enlarging and reducing device 200 capable of enlarging and reducing the formed angle formed between the below-knee member 110 and the above-knee member 120, a mechanical stop mechanism 150 that mechanically limits a range of change in the formed angle formed between the below-knee member 110 and the above-knee member 120, a buffer mechanism 160 that buffers an impact caused by the mechanical stop mechanism 150, a sensor device 270, and a battery B that supplies power to the enlarging and reducing device 200 and the like.

The above-knee member 120 includes an adapter 121 coupled to a socket which is not shown, and an above-knee base portion 126 including an upper wall 125 to which the adapter 121 is attached. The socket is a joint member provided on the thigh, and the above-knee member 120 is integrated with the thigh by coupling the adapter 121 to the socket.

The below-knee member 110 includes a box-shaped main frame 111 with open upper portion and rear portion, side covers 112 that cover both left and right side surfaces of the main frame 111, a detachable rear cover 113 that covers a rear opening of the main frame 111 in an openable and closable manner, and an adapter 122 attached to a lower surface of the main frame 111.

The above-knee member 120 is provided on the upper portion of the main frame 111 of the below-knee member 110 via a coupling axis 135 that constitutes the knee joint mechanism 130, and a leg 114 extending downward is coupled to the adapter 122 of the main frame 111.

An enlarging and reducing device 200 capable of enlarging and reducing the formed angle formed between the above-knee member 110 and the below-knee member 120 is provided in a space formed by the below-knee member 120 and the above-knee member 110. The enlarging and reducing device 200 is an expansion-contraction device 140 capable of enlarging and reducing the formed angle formed between the below-knee member 110 and the above-knee member 120 by expansion and contraction. The expansion-contraction device 140 extends in the upper-lower direction, which will be described later in detail, and is mechanically connected to the above-knee member 120 on one side in the extending direction and mechanically connected to the below-knee member 110 on the other side in the extending direction. The term "mechanically connected" is a concept that includes a configuration of direct connection and a configuration of connection via another member.

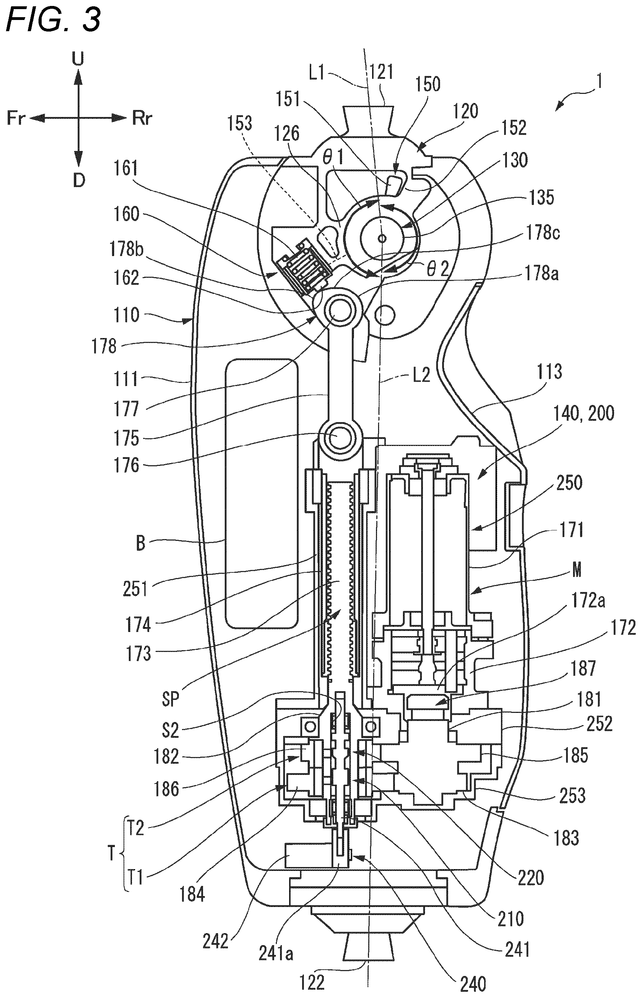

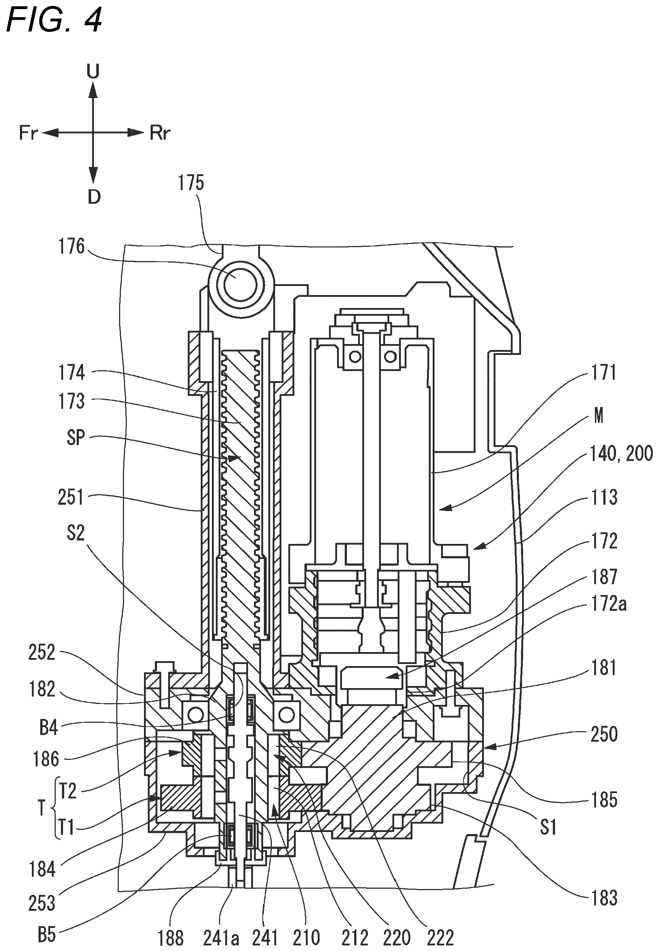

As shown in FIGs. 3 and 4, the expansion-contraction device 140 includes: a motor M that outputs rotational power, a transmission T that transmits the power of the motor M, a spindle unit SP that is connected to the transmission T in a manner of being capable of transmitting power and converts the rotational power output from the transmission T into translational motion (expanding and contracting motion), a first connection and disconnection mechanism 210 and a second connection and disconnection mechanism 220 which are provided in the transmission T, an operation mechanism 240 for switching between the first connection and disconnection mechanism 210 and the second connection and disconnection mechanism 220, and a control unit 10 that controls the motor M, and controls the first connection and disconnection mechanism 210 and the second connection and disconnection mechanism 220 by operating the operation mechanism 240.

The motor M is, for example, a permanent-magnet-type electric motor, and is disposed on the rear side and upper side of the transmission T, and the spindle unit SP is disposed on the front side and upper side of the transmission T. The spindle unit SP is disposed on a side opposite to the motor M with respect to the transmission T on a transmission path of the power. The motor M is a motor with a built-in gear mechanism, including a motor body 171 and a gear mechanism unit 172 that decelerates output rotation of the motor body 171. The spindle unit SP includes a spindle 173 formed with a male screw and a sleeve 174 formed with a female screw, and rotation of the spindle 173 causes the sleeve 174 to perform translational motion along an axis of the spindle 173.

Specifically, the spindle 173 rotates after receiving the rotational power of the motor M transmitted by the transmission T. On the other hand, the sleeve 174 is non-rotatably and movably in the upper-lower direction supported by a unit case 250. Therefore, when the spindle 173 rotates to one side after receiving the rotational power of the motor M transmitted by the transmission T, the sleeve 174 is translated away from the transmission T, and when the spindle 173 rotates to the other side, the sleeve 174 is translated in a direction of approaching the transmission T. The translation operation of the sleeve 174 away from the transmission T may be referred to as an expanding operation of the spindle unit SP, and conversely, the translation operation of the sleeve 174 approaching the transmission T may be referred to as a contracting operation of the spindle unit SP.

That is, a distance between the sleeve 174 and the transmission T increases or decreases depending on a rotation direction of the spindle 173. An upper end of the sleeve 174 is coupled to the above-knee member 120 via a link member 175. As the distance between the sleeve 174 and the transmission T increases or decreases depending on the rotation direction of the spindle 173, the below-knee member 110 and the above-knee member 120 rotate around the coupling axis 135. Accordingly, the formed angle formed between the above-knee member 120 and the below-knee member 110 changes.

Here, the formed angle formed between the above-knee member 120 and the below-knee member 110 is an angle defined by a first virtual line L1 connecting a center of the coupling axis 135 of the knee joint mechanism 130 and the adapter 121 of the above-knee member 120, and a second virtual line L2 extending downward in a vertical direction through the center of the coupling axis 135 of the knee joint mechanism 130 and the below-knee member 110. Among angles formed between the below-knee member 110 and the above-knee member 120 at the coupling axis 135 of the knee joint mechanism 130 as a center, an angle on one side in one circumference is defined as a first formed angle $\theta1$, and an angle on the other side in the circumference is defined as a second formed angle $\theta2$. When a smaller minimum angle, of the first formed angle $\theta1$ and the second formed angle $\theta2$, formed in a range of relative movement between the below-knee member 110 and the above-knee member 120 is the second formed angle $\theta2$, an angle (knee-back angle) formed on a back side of the knee of the user of the electric prosthetic leg 1 is defined as the second formed angle $\theta2$. The first formed angle $\theta1$ takes a value of about 175 [deg] to 300 [deg], and the second formed angle $\theta2$ takes a value of about 60 [deg] to 185 [deg].

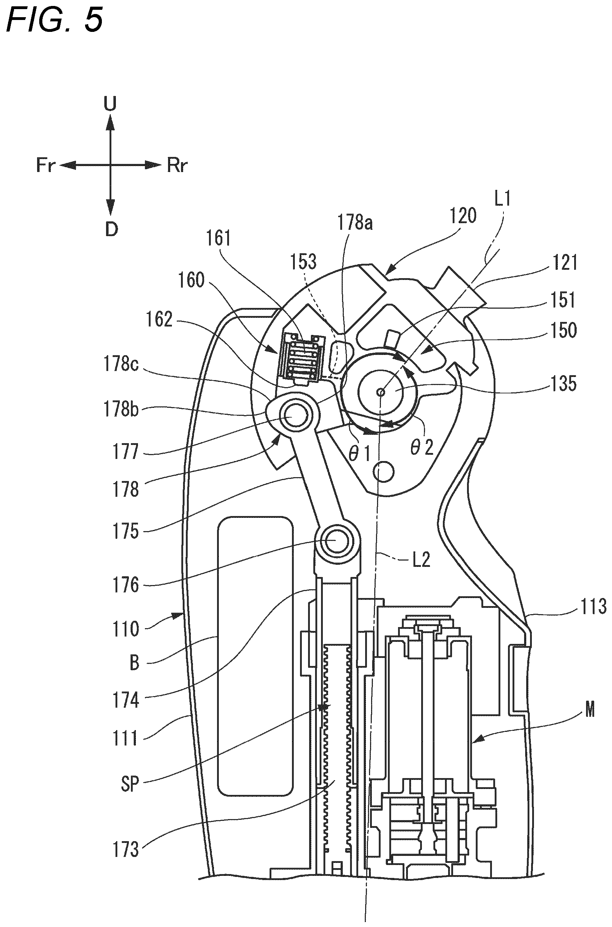

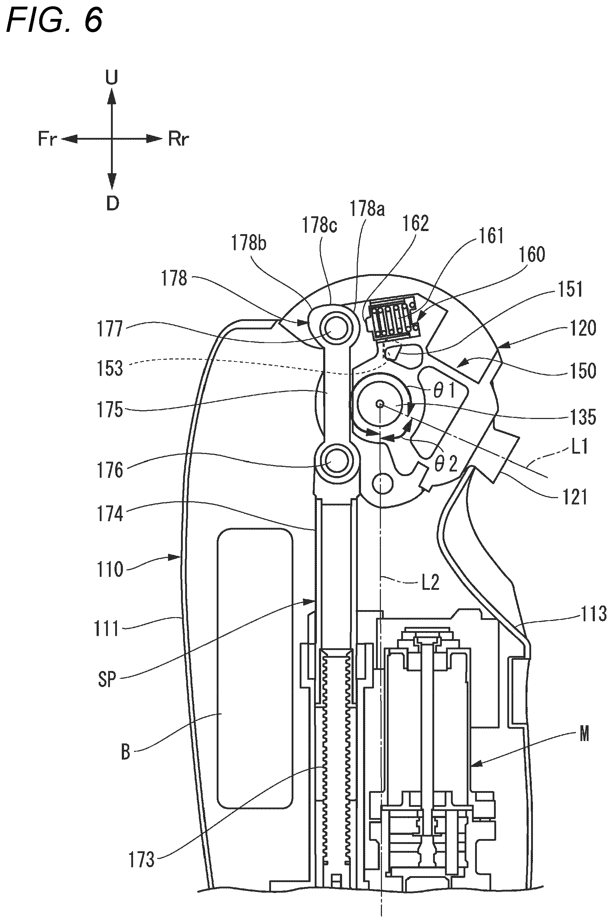

FIG. 3 shows a stretched state of the knee joint mechanism 130, in which the first formed angle $\theta1$ is about 175 [deg], and the second formed angle $\theta2$ is about 185 [deg]. FIG. 5 is a cross-sectional view of a main part showing a bent state of the electric prosthetic leg 1, in which the first formed angle $\theta1$ is about 240 [deg] and the second formed angle $\theta2$ is about 120 [deg]. FIG. 6 is a cross-sectional view of a main part showing a maximum bent state of the electric prosthetic leg 1, in which the first formed angle $\theta1$ is about 300 [deg] and the second formed angle $\theta2$ is about 60 [deg].

The enlarging and reducing device 200 of the present embodiment causes the expansion-contraction device 140 to expand and contract by converting the rotation motion into the expanding and contracting motion by the spindle unit SP of the expansion-contraction device 140, thereby enlarging and reducing the formed angle formed between the below-knee member 110 and the above-knee member 120. However, instead of a portion that expands and contracts (moves) as the expansion-contraction device 140 (spindle unit SP), a gear meshing mechanism (or the like) may be provided between the below-knee member 110 and the above-knee member 120 to enlarge and reduce the formed angle formed between the below-knee member 110 and the above-knee member 120.

Returning to FIGs. 3 and 4, the transmission T includes a first transmission mechanism T1 that includes a first transmission unit transmitting the power of the motor M to the spindle unit SP at a first transmission ratio, and a second transmission mechanism T2 that includes a second transmission unit transmitting the power of the motor M to the spindle unit SP at a second transmission ratio, which is different from the first transmission ratio. The first transmission mechanism T1 and the second transmission mechanism T2 are switched between a power disconnection state and a power connection state by the connection and disconnection mechanisms 210 and 220.

According to such a transmission T, by providing two power transmission paths with different transmission ratios, it is possible to switch an operating speed and generated power for stretching and bending in the knee joint mechanism 130. As long as the first transmission ratio is different from the second transmission ratio, one of the first transmission mechanism T1 and the second transmission mechanism T2 may be a speed reduction mechanism and the other may be a speed increasing mechanism, or one may be a constant speed mechanism and the other may be a speed reduction mechanism or a speed increasing mechanism, or both may be speed reduction mechanisms, or both may be speed increasing mechanisms.

The first transmission ratio is a ratio of a post-transmission rotation speed, which is a rotation speed on a motor M opposite-side (spindle unit SP side) of the first transmission unit in the first transmission mechanism T1, with respect to a pre-transmission rotation speed, which is a rotation speed on a motor M side of the first transmission unit in the first transmission mechanism T1. The second transmission ratio is a ratio of a post-transmission rotation speed, which is a rotation speed on a motor M opposite-side (spindle unit SP side) of a second transmission unit in the second transmission mechanism T2, with respect to a pre-transmission rotation speed, which is a rotation speed on a motor M side of the second transmission unit in the second transmission mechanism T2.

For example, when the first transmission ratio of the first transmission mechanism T1 is smaller than 1, the rotation speed on the motor M opposite-side (spindle unit SP side) becomes lower than the rotation speed on the motor M side, and a torque increases. When the second transmission ratio of the second transmission mechanism T2 is larger than 1, the rotation speed on the motor M opposite-side (spindle unit SP side) becomes higher than the rotation speed on the motor M side, and a torque decreases. In the present embodiment, the first transmission ratio is set to be smaller than 1 and the second transmission ratio is set to be larger than 1, and the first transmission mechanism T1 is disposed below the second transmission mechanism T2.

The first transmission mechanism T1 and the second transmission mechanism T2 include a first shaft 181 rotatably disposed on a downward extension line of an output axis 172a of the gear mechanism unit 172, and a second shaft 182 rotatably disposed on a downward extension line of the spindle 173 of the spindle unit SP. The first shaft 181 is coupled to the output axis 172a of the gear mechanism unit 172 of the motor M in a manner of being integrally rotatable, via a coupling 187 that allows an axis center error. The second shaft 182 is connected to the spindle 173 of the spindle unit SP in a manner of being integrally rotatable. Although the second shaft 182 of the present embodiment is integrated with the spindle 173 of the spindle unit SP, the second shaft 182 may be coupled to the spindle 173 of the spindle unit SP by spline fitting or using a coupling.

The first transmission mechanism T1 includes the first transmission unit including a first drive gear 183 and a first driven gear 184 that mesh with each other. The first drive gear 183 is supported by the first shaft 181 in a manner of being integrally rotatable, and the first driven gear 184 is supported by the second shaft 182 in a manner of being relatively rotatable. The first driven gear 184 and the second shaft 182 have the same rotation axis. The first transmission mechanism T1 of the present embodiment is a deceleration transmission mechanism in which the first drive gear 183 has a diameter smaller than that of the first driven gear 184, and can cause the spindle unit SP to expand and contract at low speed and high torque.

The second transmission mechanism T2 includes the second transmission unit including a second drive gear 185 and a second driven gear 186 that mesh with each other. The second drive gear 185 is supported by the first shaft 181 in a manner of being integrally rotatable, and the second driven gear 186 is supported by the second shaft 182 in a manner of being relatively rotatable. The second driven gear 186 and the second shaft 182 have the same rotation axis. The second transmission mechanism T2 of the present embodiment is an acceleration transmission mechanism in which the second drive gear 185 has a diameter larger than that of the second driven gear 186, and can cause the spindle unit SP to expand and contract at high speed and low torque. In the present embodiment, the second transmission mechanism T2 is disposed above the first transmission mechanism T1, but the second transmission mechanism T2 may be disposed below the first transmission mechanism T1. Although the first shaft 181 and the second shaft 182 of the present embodiment are integrally formed from the beginning, the first shaft 181 and the second shaft 182 may be integrally coupled (combined) after upper and lower gear support portions are formed as separate bodies.

The first connection and disconnection mechanism 210 includes a connection and disconnection unit 212 provided between the first driven gear 184 and the second shaft 182. The second connection and disconnection mechanism 220 includes a connection and disconnection unit 222 provided between the second driven gear 186 and the second shaft 182. These connection and disconnection units 212 and 222 have a common configuration, and are configured to be switched between a disconnection state where power transmission is disconnected, and a power transmittable state where rotational power in both one direction and the other direction can be transmitted. Details of the connection and disconnection units 212 and 222 will be described later.

The operation mechanism 240 includes an operation rod 241 configured to intermittently operate the connection and disconnection units 212 and 222, and a servo motor 242 that linearly moves the operation rod 241.

The second shaft 182 is a hollow shaft having an internal space S2 extending in a rotation axis direction (also referred to as the upper-lower direction), and the operation rod 241 is disposed in the internal space S2. The operation rod 241 is provided with a rack 241a at a lower end exposed from the internal space S2. The operation rod 241 is supported by bearings B4 and B5 disposed in the internal space S2 in a manner of not being relatively rotatable with respect to the rack 241a and being capable of integrally advancing and retracting with the rack 241a in the rotation axis direction. A lid member 188 having an insertion hole through which the operation rod 241 is inserted is screwed to a lower end of the second shaft 182. The lid member 188 prevents foreign matters from entering the internal space S2 and facilitates replacement of the operation rod 241. A pinion 243 provided on an output axis 242a of the servo motor 242 meshes with the rack 241a, and a position of the operation rod 241 in the upper-lower direction is switched according to the drive of the servo motor 242. Small-diameter portions 241b1 and 241b2 and large-diameter portions 241c1 to 241c3, which will be described later, are formed on an outer peripheral portion of the operation rod 241, and the small-diameter portions 241b1 and 241b2 and the large-diameter portions 241c1 to 241c3 intermittently operate the connection and disconnection units 212 and 222 according to the position of the operation rod 241. Details of the operation mechanism 240 will be described later.

As shown in FIGs. 3 to 5, the unit case 250 includes an upper case 251, a middle case 252, and a lower case 253. The upper case 251, the middle case 252, and the lower case 253 are formed separately from each other.

The upper case 251 accommodates the spindle unit SP.

A space S1 defined by the middle case 252 and the lower case 253 accommodates the second drive gear 185, the second driven gear 186, the first drive gear 183, the first driven gear 184, the connection and disconnection units 212 and 222, and a part of the operation mechanism 240.

With the three-stage structure of the upper case 251, the middle case 252, and the lower case 253, the unit case 250 not only can house the transmission T and the spindle unit SP, but also can unitize the expansion-contraction device 140 including the motor M.

Further, the unit case 250 is attached to the main frame 111 via a bracket which is not shown.

As shown in FIGs. 3, 5, and 6, the mechanical stop mechanism 150 includes a stopper member 151 provided on the below-knee member 110, and a first contact portion 152 and a second contact portion 153 provided on the above-knee base portion 126 of the above-knee member 120. In a state shown in FIG. 3 (the second formed angle θ2 is about 185 [deg]), the first contact portion 152 comes into contact with the stopper member 151, thereby restricting the knee joint mechanism 130 from bending to an opposite direction. Further, in a state shown in FIG. 6 (the second formed angle θ2 is about 60 [deg]), the second contact portion 153 comes into contact with the stopper member 151, thereby restricting the knee joint mechanism 130 from being further bent from the maximum bent state. In walking with the electric prosthetic leg 1, the maximum bent state shown in FIG. 6 does not occur.

The buffer mechanism 160 is provided on an above-knee member 120 side, and includes a pressing portion 162 capable of pressing an upper end of the link member 175 by a biasing force of a spring 161 (for example, a compression coil spring). A lower end of the link member 175 is rotatably coupled to the sleeve 174 of the spindle unit SP via a first pivoting portion 176, and the upper end of the link member 175 is rotatably coupled to the above-knee member 120 via a second pivoting portion 177. A cam portion 178 is formed at the upper end of the link member 175. The cam portion 178 includes a small-diameter outer peripheral portion 178a having a small diameter and centered on the second pivoting portion 177, a large-diameter outer peripheral portion 178b with a long distance from the second pivoting portion 177, and a coupling outer peripheral portion 178c that couples the small-diameter outer peripheral portion 178a and the large-diameter outer peripheral portion 178b without any step.

As shown in FIGs. 5 and 6, in a state where the knee joint mechanism 130 is bent, the pressing portion 162 faces the small-diameter outer peripheral portion 178a of the cam portion 178, and thus the pressing portion 162 and the cam portion 178 are separated from each other. As shown in FIG. 3, when the knee joint mechanism 130 is stretched in response to a contracting operation of the spindle unit SP and approaches a mechanical stop position on a stretching side, as a facing position between the pressing portion 162 and the cam portion 178 moves from the coupling outer peripheral portion 178c to the large-diameter outer peripheral portion 178b, the cam portion 178 comes into contact with the pressing portion 162 and the large-diameter outer peripheral portion 178b pushes the pressing portion 162 against the biasing force of the spring 161. In other words, the cam portion 178 is pressed in a return direction by the biasing force of the spring 161. Accordingly, the biasing force of the spring 161 acts as resistance, and an impact when the first contact portion 152 comes into contact with the stopper member 151 is buffered.

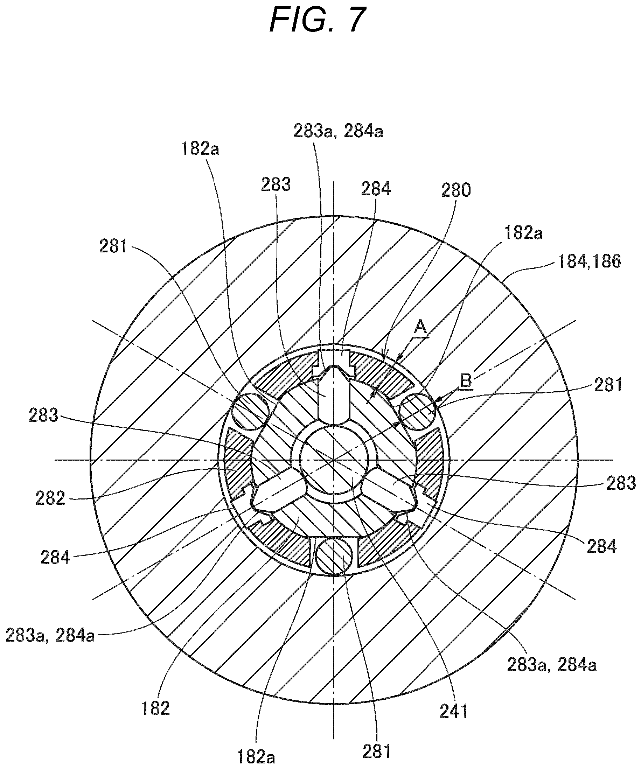

Next, details of the connection and disconnection units 212 and 222 and the operation mechanism 240 will be described with reference to FIG. 7 and subsequent drawings.

The connection and disconnection units 212 and 222 have a common configuration, and are configured to be switched between the disconnection state where the power transmission is disconnected, and the power transmittable state where the rotational power in both one direction and the other direction can be transmitted. Each of the connection and disconnection units 212 and 222 of the present embodiment is configured using a two-way clutch 280 with a forced free function, as shown in FIG. 7. The two-way clutch 280 includes a plurality of (three in the present embodiments) rollers 281 that are arranged between an outer peripheral surface portion of the second shaft 182 and inner peripheral surface portions of the gears 184 and 186, a retainer 282 that holds the plurality of rollers 281 at predetermined intervals, a plurality of (three in the present embodiment) pins 283 that penetrate the second shaft 182 in a radial direction and are operated by the operation mechanism 240 to a forced free position and a forced free release position, and a plurality of (three in the present embodiment) guides 284 that are provided in the retainer 282 and defines a relative rotational position of the retainer 282 with respect to the second shaft 182 when the pins 283 are in the forced free position. The rollers 281 may be balls or sprags.

A distance A in the radial direction between the outer peripheral surface portion of the second shaft 182 and the inner peripheral surface portions of the gears 184 and 186 is smaller than a diameter B of each of the rollers 281. Further, flat portions 182a are formed on an outer peripheral portion of the second shaft 182 at predetermined intervals in a circumferential direction, and on a center side in the circumferential direction of each of the flat portions 182a, the distance A is larger than the diameter B.

In other words, when the rollers 281 are held at center portions of the flat portions 182a in the circumferential direction, the rollers 281 do not mesh with the outer peripheral surface portion of the second shaft 182 and the inner peripheral surface portions of the gears 184 and 186 (non-engagement state), and relative rotation between the second shaft 182 and the gears 184 and 186 is allowed (forced free state).

On the other hand, when the rollers 281 are allowed to move in the circumferential direction relative to the second shaft 182, the rollers 281 mesh with the outer peripheral surface portion of the second shaft 182 and the inner peripheral surface portions of the gears 184 and 186 (engagement state), and the second shaft 182 and the gears 184 and 186 are connected in a manner of being rotatable integrally in two directions (forced free release state).

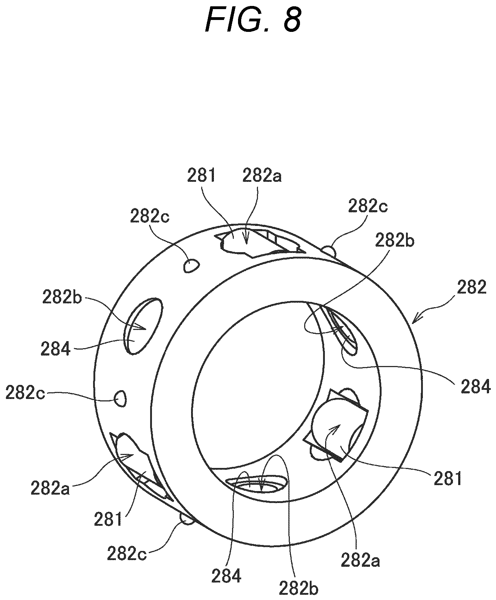

As shown in FIG. 8, the retainer 282 includes a plurality of roller holding portions 282a that are ring-shaped and capable of relatively rotating with respect to the second shaft 182 and the gears 184 and 186, and that hold the rollers 281, and a plurality of guide holding portions 282b that hold the guides 284.

In an outer peripheral surface of the retainer 282, a plurality of rubber balls 282c are embedded at predetermined intervals in the circumferential direction. These rubber balls 282c prevent unintended idling in the forced free release state by generating moderate friction between the gears 184 and 186 and the retainer 282. The members for generating friction between the gears 184 and 186 and the retainer 282 are not limited to the rubber balls 282c, and may be O-rings.

Returning to FIG. 7, each of the pins 283 includes a conical protrusion portion 283a on an outer end in the radial direction, and each of the guides 284 includes, on an inner end surface thereof in the radial direction, a conical recess portion 284a that fits to (engages with) the protrusion portion 283a. When the protrusion portion 283a of the pin 283 fits to the recess portion 284a of the guide 284, a relative rotation position of the retainer 282 with respect to the second shaft 182 is positioned at a predetermined position where the retainer 282 is in the forced free state by a guiding effect of the pins 283 and the guides 284.

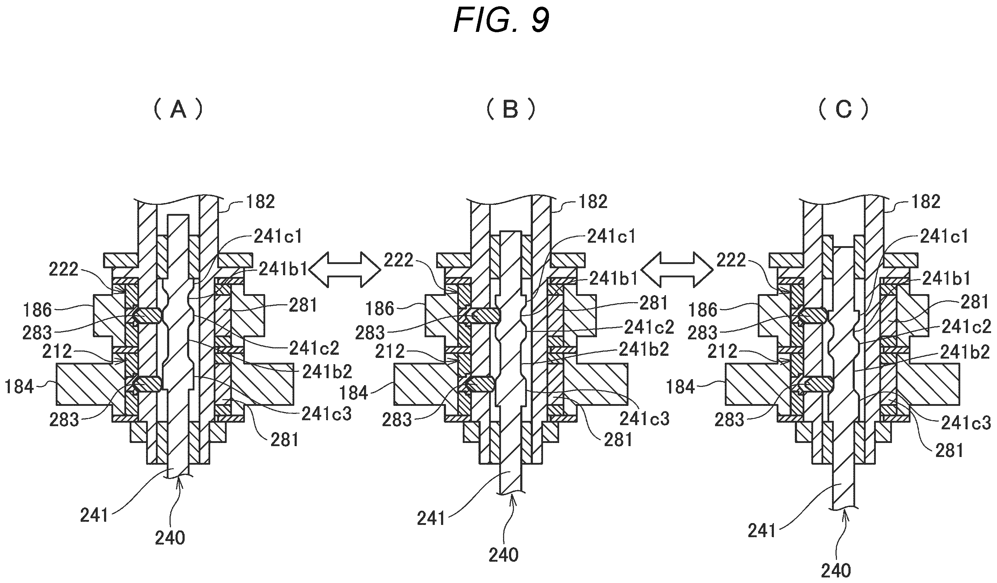

As shown in FIG. 9, the operation rod 241 includes, in the order from the upper side, a first large-diameter portion 241c1, a first small-diameter portion 241b1, a second large-diameter portion 241c2, a second small-diameter portion 241b2, and a third large-diameter portion 241c3 formed with predetermined lengths and intervals therebetween. The operation rod 241 is provided to be capable of simultaneously controlling the two connection and disconnection units 212 and 222, but may be provided separately for each of the connection and disconnection units 212 and 222.

In the following description, operation of the operation mechanism 240 that simultaneously controls the connection and disconnection units 212 and 222 will be described with reference to FIG. 9.

As shown in FIG. 9, the connection and disconnection units 212 and 222 are switched between the forced free state (hereinafter referred to as an off state as appropriate) and the forced free release state (hereinafter referred to as an on state as appropriate) by the operation mechanism 240.

When the operation rod 241 of the operation mechanism 240 is in an upper position shown in (A) of FIG. 9, the third large-diameter portion 241c3 pushes out the pin 283 of the connection and disconnection unit 222 in an outer diameter direction while the second large-diameter portion 241c2 pushes out the pin 283 of the connection and disconnection unit 212 in the outer diameter direction, thus setting the connection and disconnection unit 212 and the connection and disconnection unit 222 to the off state.

Further, when the operation rod 241 of the operation mechanism 240 is in a middle position shown in (B) of FIG. 9, the third large-diameter portion 241c3 pushes out the pin 283 of the connection and disconnection unit 212 in the outer diameter direction while the first small-diameter portion 241b1 allows the pin 283 of the connection and disconnection unit 222 to return in an inner diameter direction, thereby setting the connection and disconnection unit 222 to the on state and the connection and disconnection unit 212 to the off state.

Further, when the operation rod 241 of the operation mechanism 240 is in a lower position shown in (C) of FIG. 9, the second small-diameter portion 241b2 allows the pin 283 of the connection and disconnection unit 212 to return in the inner diameter direction while the first large-diameter portion 241c1 pushes out the pin 283 of the connection and disconnection unit 222 in the outer diameter direction, thus setting the connection and disconnection unit 222 to the off state and the connection and disconnection unit 212 to the on state.

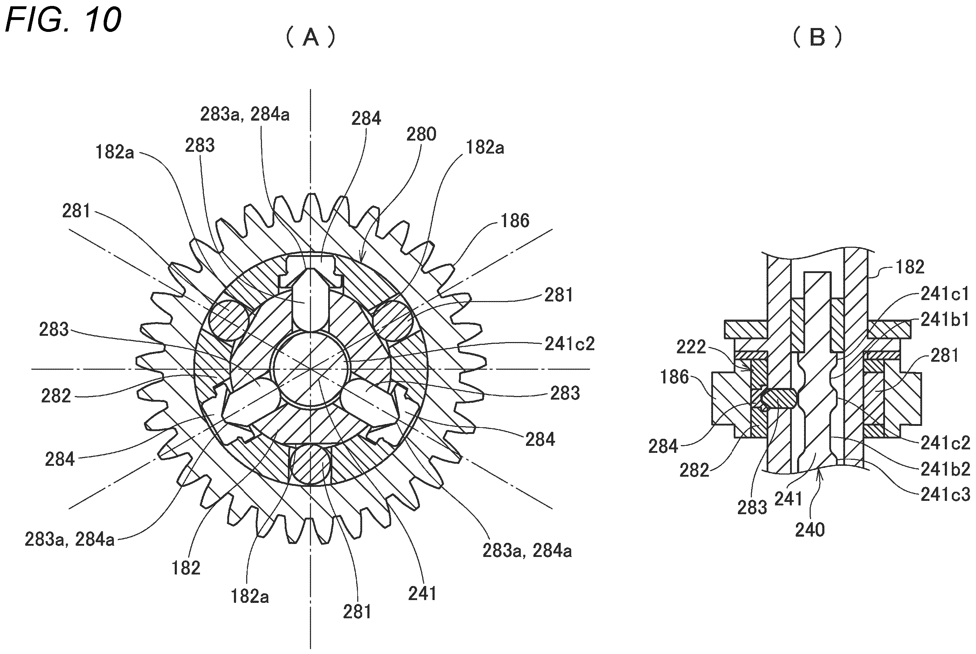

Next, the operation of the two-way clutch 280 will be described with reference to FIGs. 10 to 14, taking the connection and disconnection unit 222 as an example. In the following example, a case of a transition from (A) to (B) to (C) in FIG. 9 in the connection and disconnection unit 222 will be described as an example.

As shown in (A) and (B) of FIG. 10, in a state where the second large-diameter portion 241c2 of the operation rod 241 pushes out the pin 283 of the connection and disconnection unit 222 in the outer diameter direction, the protrusion portion 283a of the pin 283 fits to the recess portion 284a of the guide 284, and the relative rotational position of the retainer 282 with respect to the second shaft 182 is fixed at a predetermined position. In this state, since the rollers 281 are held at the center portion of the flat portion 182a in the circumferential direction, the rollers 281 do not mesh with the outer peripheral surface portion of the second shaft 182 and the inner peripheral surface portion of the second driven gear 186, and the state becomes the off state where the relative rotation between the second shaft 182 and the second driven gear 186 is allowed.

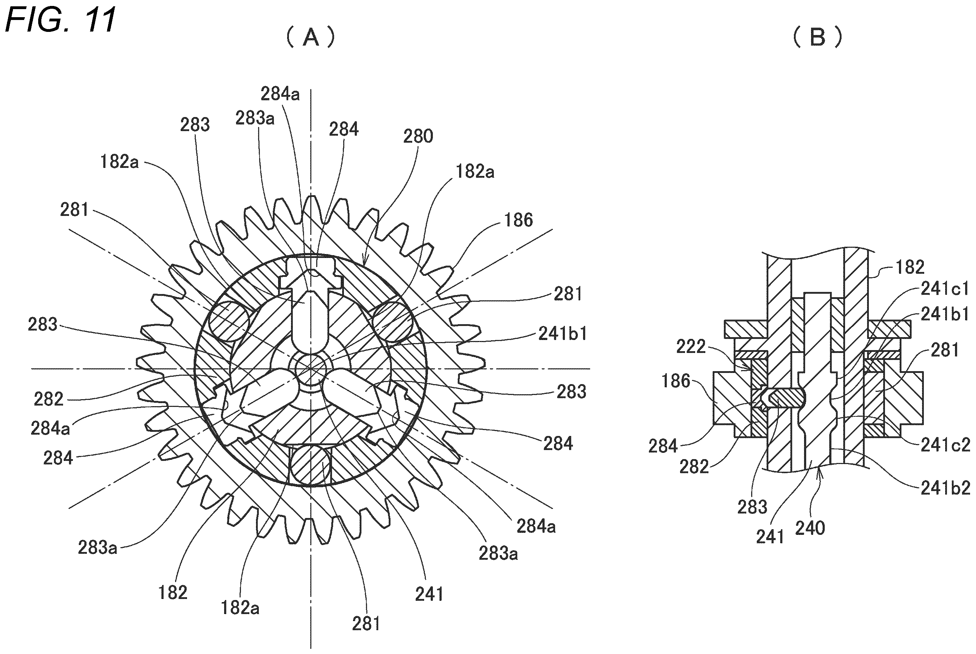

(A) and (B) of FIG. 11 show a state where the operation rod 241 moves from the position where the second large-diameter portion 241c2 pushes out the pin 283 of the connection and disconnection unit 222 in the outer diameter direction to the position where the first small-diameter portion 241b1 allows the pin 283 to return in the inner diameter direction. In FIG. 11, the pin 283 is moved in the inner diameter direction, but actually, at a timing when the relative rotation between the second shaft 182 and the second driven gear 186 occurs, the guides 284 of the retainer 282 that rotate together with the second driven gear 186 pushes the pin 283 back in the inner diameter direction on an inclined surface of the recess portion 284a.

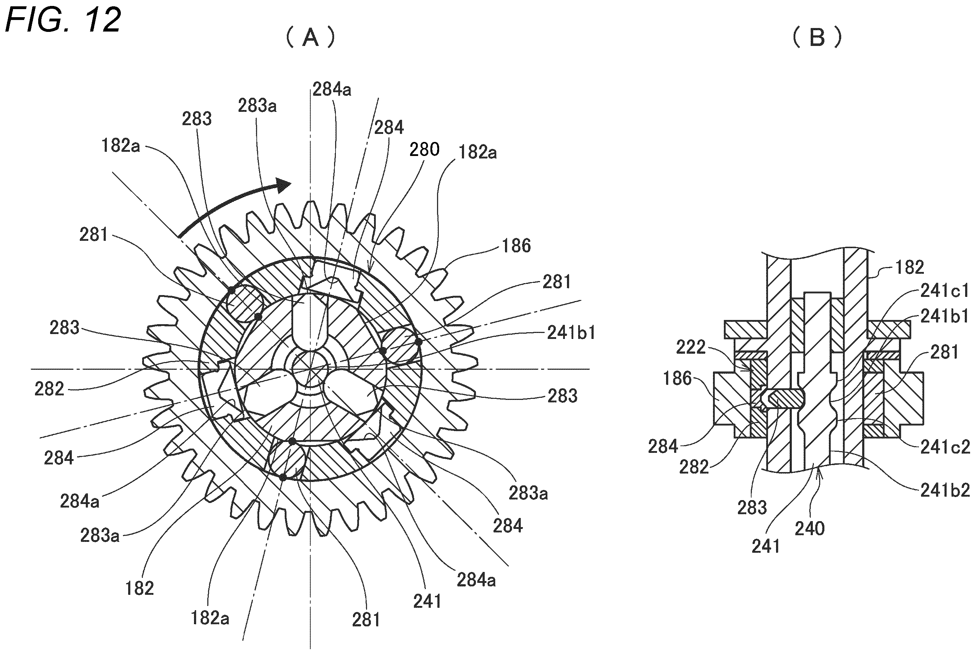

As shown in (A) and (B) of FIG. 12, in a state where the pin 283 is allowed to return in the inner diameter direction, when relative rotation occurs between the second shaft 182 and the second driven gear 186 in a normal rotation direction shown by an arrow in the drawing, the retainer 282 that rotates together with the second driven gear 186 moves the rollers 281 in the normal rotation direction with respect to the second shaft 182. Accordingly, the rollers 281 mesh with the outer peripheral surface portion of the second shaft 182 and the inner peripheral surface portion of the second driven gear 186, and a normal-rotation on state is created in which the second shaft 182 and the second driven gear 186 are rotated integrally in the normal rotation direction.

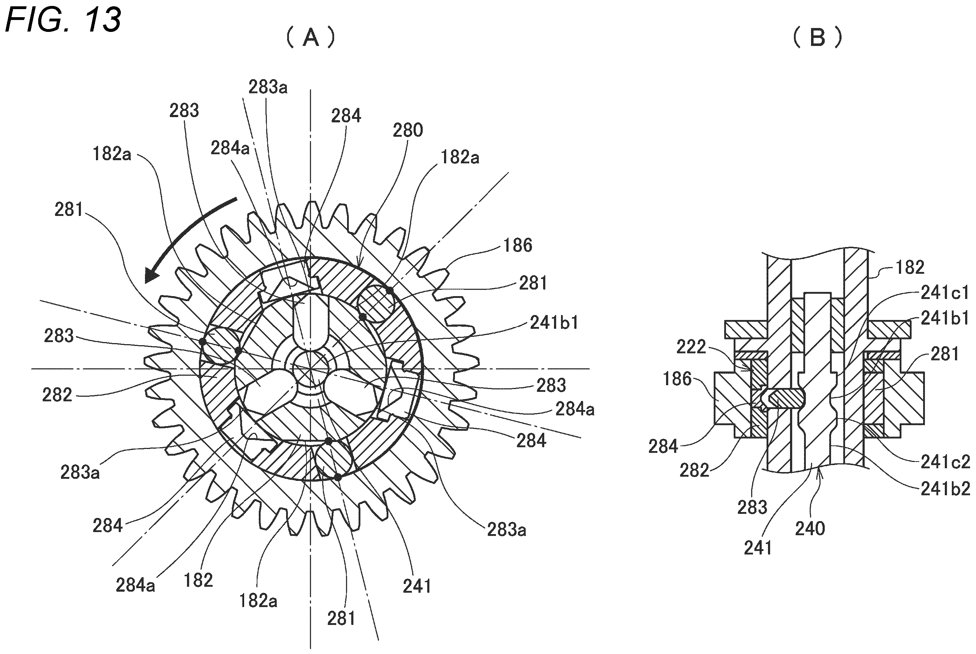

As shown in (A) and (B) of FIG. 13, in a state where the pin 283 is allowed to return in the inner diameter direction, when relative rotation occurs between the second shaft 182 and the second driven gear 186 in a reverse rotation direction shown by an arrow in the drawing, the retainer 282 that rotates together with the second driven gear 186 moves the rollers 281 in the reverse rotation direction with respect to the second shaft 182. Accordingly, the rollers 281 mesh with the outer peripheral surface portion of the second shaft 182 and the inner peripheral surface portion of the second driven gear 186, and a reverse-rotation on state is created in which the second shaft 182 and the second driven gear 186 are rotated integrally in the reverse rotation direction.

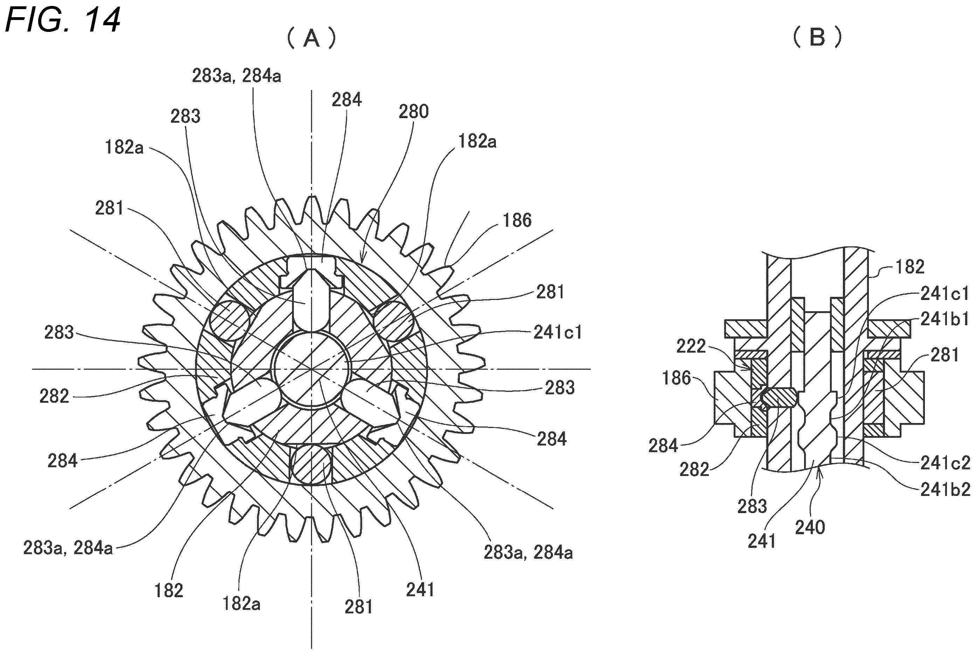

As shown in (A) and (B) of FIG. 14, when the operation rod 241 moves from a position where the first small-diameter portion 241b1 allows the pin 283 of the connection and disconnection unit 222 to return in the inner diameter direction, to a position where the first large-diameter portion 241c1 pushes out the pin 283 in the outer diameter direction, the protrusion portion 283a of the pin 283 fits to the recess portion 284a of the guide 284, and the relative rotation position of the retainer 282 with respect to the second shaft 182 is fixed at a predetermined position, by the guiding effect of the pin 283 and the guide 284. In this state, since the rollers 281 are held at the center portion of the flat portion 182a in the circumferential direction, the rollers 281 do not mesh with the outer peripheral surface portion of the second shaft 182 and the inner peripheral surface portion of the second driven gear 186, and the state becomes the off state where the relative rotation between the second shaft 182 and the second driven gear 186 is allowed.

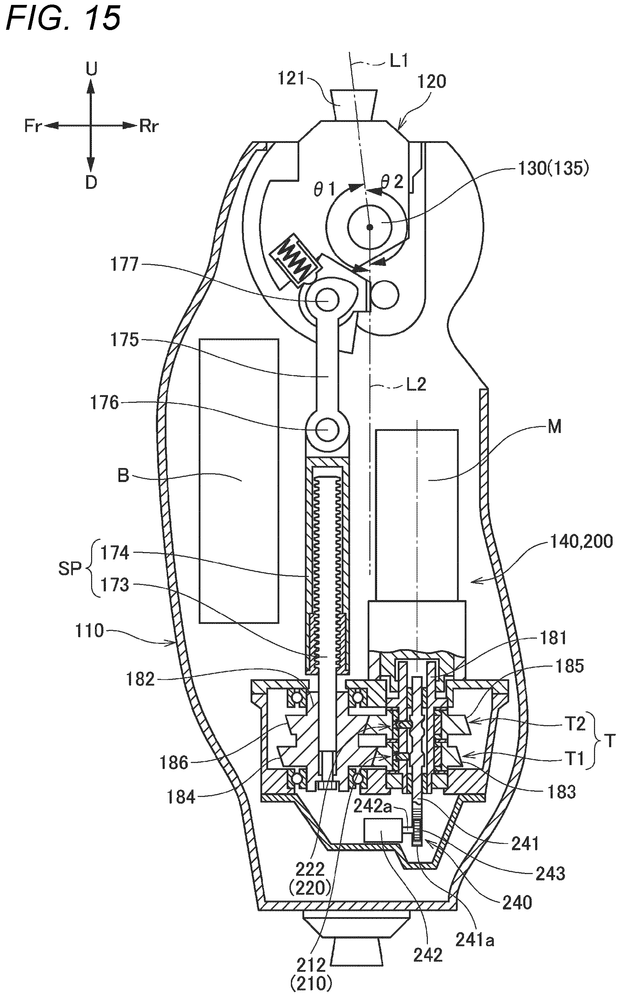

In the above embodiment, the connection and disconnection units 212 and 222 and the operation mechanism 240 are provided on a second shaft 182 side, but may be provided on a first shaft 181 side as in a second embodiment shown in FIG. 15. That is, in the electric prosthetic leg 1 of the second embodiment, the connection and disconnection unit 212 of the first connection and disconnection mechanism 210 is provided between the first drive gear 183 and the first shaft 181, and the connection and disconnection unit 222 of the second connection and disconnection mechanism 220 is provided between the second drive gear 185 and the first shaft 181. Since other configurations are substantially the same as or similar to those of the first embodiment, the following description will be made by taking the electric prosthetic leg 1 of the first embodiment as an example.

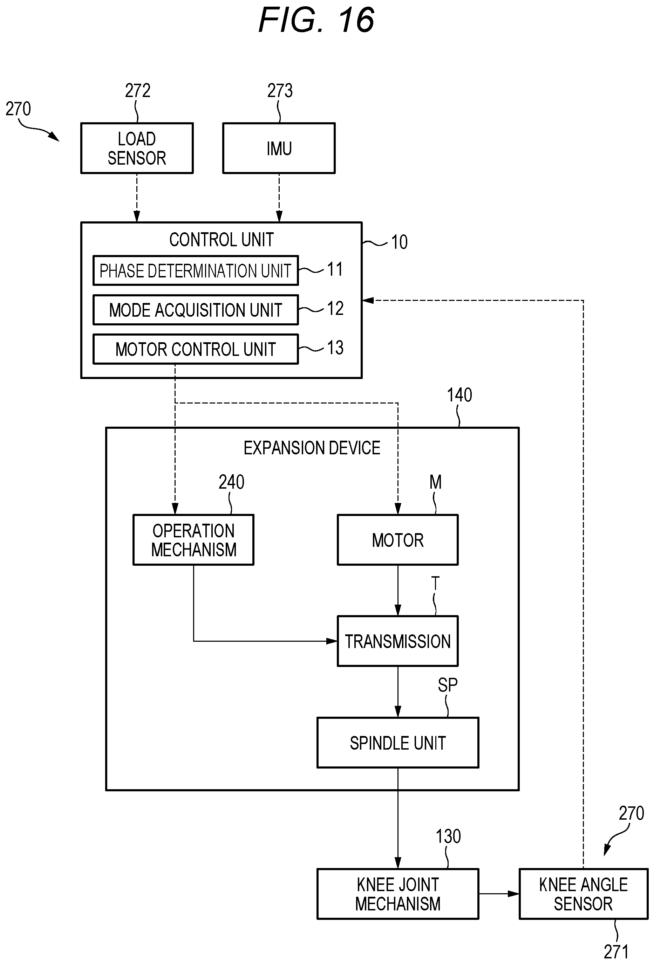

As shown in FIG. 16, the sensor device 270 includes a knee angle sensor 271 provided in the knee joint mechanism 130 (coupling axis 135), a load sensor 272 incorporated in the adapter 122, and an inertial measurement unit (IMU) 273 mounted on a substrate (not shown) disposed in the vicinity of the motor M.

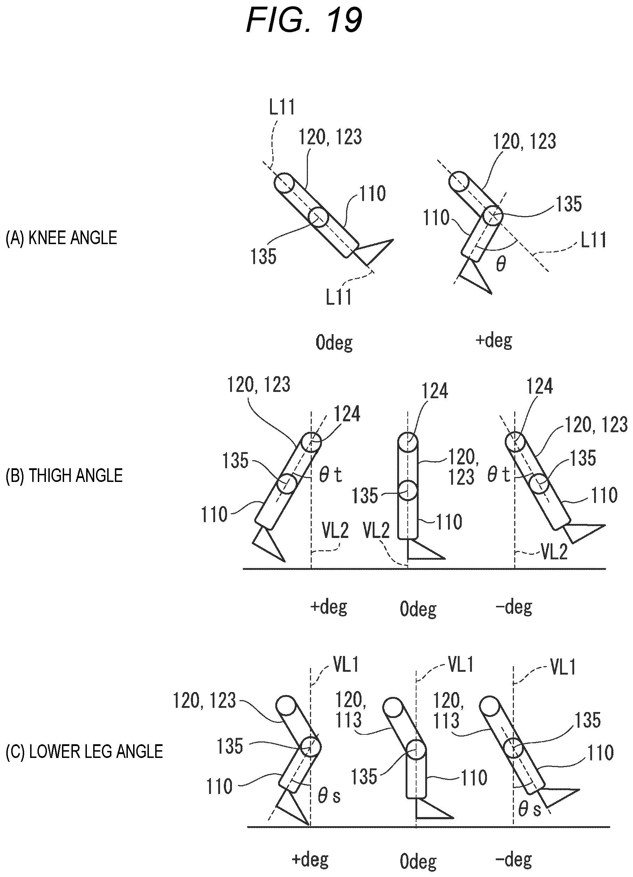

The knee angle sensor 271 detects a knee angle ($\theta$ [deg]). As shown in (A) of FIG. 19, the knee angle ($\theta$ [deg]) is a formed angle formed by an extension line L11 of the above-knee member 120 passing through the coupling axis 135 and the below-knee member 110. In other words, the knee angle $\theta$ is an angle obtained by subtracting the second formed angle $\theta 2$ from 180 [deg], and is a supplementary angle of the second formed angle $\theta 2$. The knee angle $\theta$ takes a negative value when the below-knee member 110 is in front of the extension line L11, and the knee angle $\theta$ takes a positive value (+deg) when the below-knee member 110 is behind the extension line L11. Therefore, a possible range of the second formed angle $\theta 2$ is about 60 [deg] to 185 [deg], and a possible range of the knee angle $\theta$ is about -5 [deg] to 120 [deg].

The load sensor 272 detects a weight applied to the electric prosthetic leg 1, in other words, a load from the user of the electric prosthetic leg 1. The load sensor 272 is set such a tensile load leads to a positive value and a compression load leads to a negative value. Therefore, in a weighted state where the electric prosthetic leg 1 is in contact with the ground (hereinafter, may be referred to as a stance phase), a negative value is obtained when a weight (compression load) is applied to the electric prosthetic leg 1 from the outside, and in a non-weighted state where the electric prosthetic leg 1 is separated from the ground (hereinafter, may be referred to as a swing phase), a positive value is obtained when a tensile load is applied due to an own weight of the electric prosthetic leg 1 although no weight is received from the outside.

The IMU 273 acquires angular velocities in three axes and accelerations in the three axes. When three axes of an orthogonal coordinate system are an X-axis, a Y-axis, and a Z-axis, the IMU 273 detects an X-axis angular velocity $\omega x$ [deg/s], a Y-axis angular velocity $\omega y$ [deg/s], a Z-axis angular velocity $\omega z$ [deg/s], an X-axis acceleration Ax [m/s$^2$], a Y-axis acceleration Ay [m/s$^2$], and a Z-axis acceleration Az [m/s$^2$].

Next, the control unit 10 that controls the electric prosthetic leg 1 will be described.

The control unit 10 drives the electric prosthetic leg 1 in a stair ascending mode to be described later when the user walks up stairs, and drives the electric prosthetic leg 1 in a flat ground and stair descending mode to be described later when the user walks to advance on a flat ground or walks to ascend stairs. The control unit 10 receives information from the sensor device 270 and controls the electric prosthetic leg 1 in each mode. More specifically, the control unit 10 receives information from the knee angle sensor 271, the load sensor 272, and the IMU 273. The control unit 10 acquires the knee angle θ from the knee angle sensor 271, acquires the load from the load sensor 272, and calculates a lower leg angle θs from the angular velocities in the three axes and the accelerations in the three axes detected by the IMU 273. The control unit 10 calculates a thigh angle θt from the knee angle θ and the lower leg angle θs. In addition, the control unit 10 can calculate an angular velocity or an angular acceleration of each of the knee angle θ, the lower leg angle θs, and the thigh angle θt, accelerations of the below-knee member 110 and the above-knee member 120, and the like.

Here, as shown in (C) of FIG. 19, the lower leg angle θs is a formed angle formed by a center line extending in an extending direction of the below-knee member 110 and centered on the coupling axis 135 and a vertical line VL1 passing through the coupling axis 135. When the below-knee member 110 is in front of the vertical line VL1, the lower leg angle θs takes a negative value (-deg), and when the below-knee member 110 is behind the vertical line VL1, the lower leg angle θs takes a positive value (+deg).

Further, as shown in (B) of FIG. 19, the thigh angle θt is a formed angle formed by a vertical line VL2 passing through a hip joint 124 of a thigh 123 to which the above-knee member 120 is attached, and a center line extending in an extending direction of the above-knee member 120. When the above-knee member 120 is in front of the vertical line VL2, the thigh angle θt takes a negative value (-deg), and when the above-knee member 120 is behind the vertical line VL2, the thigh angle θt takes a positive value (+deg).

In each mode, the control unit 10 controls the operation mechanism 240 (the servo motor 242) that switches between the disconnection state and the power transmittable state of the first connection and disconnection mechanism 210 and the second connection and disconnection mechanism 220 based on the information, and controls the motor M that outputs power for stretching or bending the electric prosthetic leg 1.

Referring back to FIG. 16, the control unit 10 includes a phase determination unit 11 that determines whether the electric prosthetic leg 1 is in the stance phase or the swing phase, a mode acquisition unit 12 that predicts and acquires a transition to any one of the stair ascending mode and the flat ground and stair descending mode, and a motor control unit 13 that controls the motor M that expands and contracts the expansion-contraction device 140 and the servo motor 242 (operation mechanism 240) that drives the operation rod 241.

When the load detected by the load sensor 272 is equal to or larger than a first threshold (for example, -90 [N]), the phase determination unit 11 determines the swing phase, and when the load is equal to or less than a second threshold (for example, -110 [N]), the phase determination unit 11 determines the stance phase. Further, the phase determination unit 11 determines that the phase transitions from the stance phase to the swing phase when the load becomes equal to or larger than the first threshold (for example, -90 [N]) in a state where the stance phase is determined. On the other hand, when the load becomes equal to or less than the second threshold (for example, -110 [N]) in a state where the swing phase is determined, the transition from the swing phase to the stance phase is determined. The first threshold and the second threshold may be the same value, but are preferably different values by providing hysteresis. This can further prevent hunting.

Based on a predetermined condition, the mode acquisition unit 12 predicts and acquires a transition from the stair ascending mode to the flat ground and stair descending mode and a transition from the flat ground and stair descending mode to the stair ascending mode. Further, the mode acquisition unit 12 may acquire the mode transition by a changeover switch provided in the electric prosthetic leg 1 or an electric switch that receives a signal from a terminal device of the user, for example, a smartphone, a mobile phone, a tablet, a smart watch, or the like.

The motor control unit 13 controls the motor M to extend and contract the expansion-contraction device 140 so as to stretch or bend the electric prosthetic leg 1. Further, the motor control unit 13 controls the servo motor 242 to move the operation rod 241, so as to switch the first connection and disconnection mechanism 210 and the second connection and disconnection mechanism 220 between the disconnection state and the power transmittable state. Further, in the power transmittable state, a speed change state (hereinafter, referred to as a high-torque-side connection state) where the power transmission state is established via the first transmission mechanism T1 and a speed change state (hereinafter, referred to as a high-speed-rotation-side connection state) where the power transmission state is established via the second transmission mechanism T2 are switched.

<Stair Ascending Mode>

In the electric prosthetic leg 1 configured in this way, it is possible to smoothly ascend stairs, which has been required to be done one by one by a leg on a non-prosthetic leg side (healthy leg), with a passive prosthetic leg including a passive damper in the related art. A mode in which the user walks upstairs is the stair ascending mode.

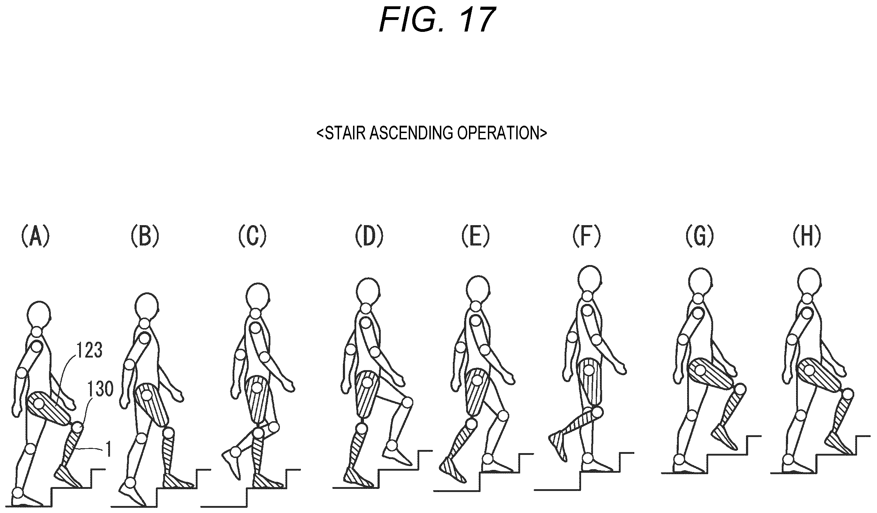

FIG. 17 is a diagram showing actions (stair ascending actions) of a human and the electric prosthetic leg when ascending stairs.

(A) to (D) of FIG. 17 are diagrams showing the stance phase, (D) and (E) of FIG. 17 are diagrams showing a transition phase from a stance to an initial swing, (E) to (G) of FIG. 17 are diagrams showing an initial swing phase, (G) of FIG. 17 is a diagram showing a transition phase from the initial swing to a terminal swing and a terminal swing phase, and (H) of FIG. 17 is a diagram showing a transition phase from the terminal swing to the stance and the stance phase.

As shown in (A) to (D) of FIG. 17, large power is required when the knee joint mechanism 130 is stretched from a bending state, in a state where a load is applied to the electric prosthetic leg 1 when moving the electric prosthetic leg 1 forward to ascending stairs.

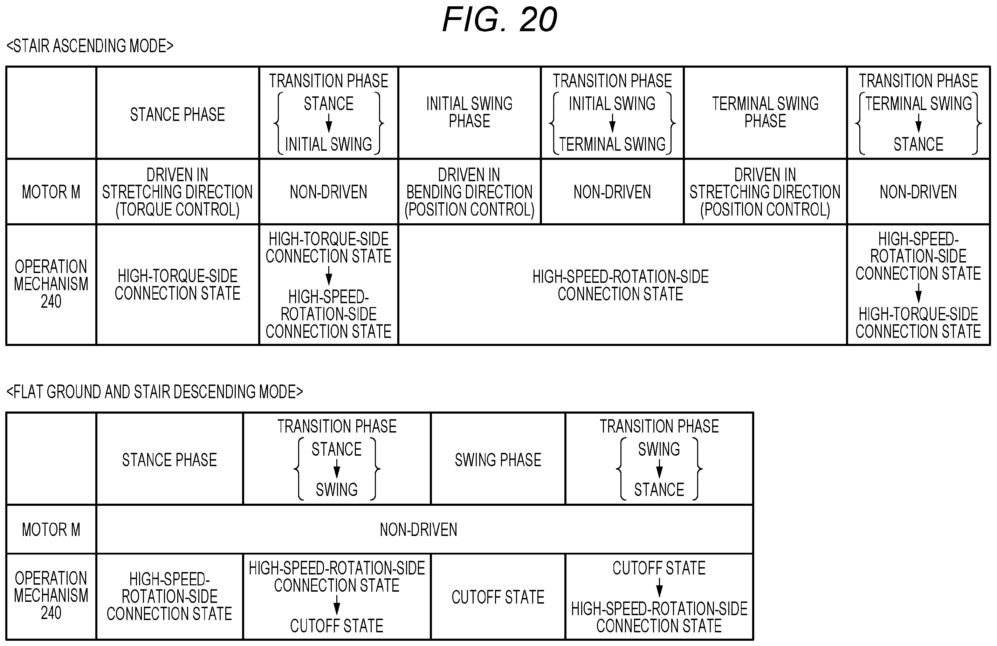

During the stance ((A) to (D) of FIG. 17), the motor control unit 13 drives the servo motor 242 to set the first connection and disconnection mechanism 210 and the second connection and disconnection mechanism 220 into a high-torque-side connection state (a stance phase in FIG. 20). In this high-torque-side connection state, when the motor M is rotated in a stretching direction, that is, in a direction of enlarging the second formed angle $\theta2$, the power of the motor M is transmitted to the first shaft 181, the first drive gear 183, the first driven gear 184, the connection and disconnection unit 212 of the first connection and disconnection mechanism 210, the second shaft 182, and the spindle unit SP. Accordingly, the sleeve 174 is expanded to be separated from the transmission T, and the above-knee member 120 to which the sleeve 174 is coupled is rotated about the coupling axis 135 with respect to the below-knee member 110 to which the transmission T is attached, and the knee joint mechanism 130 is stretched. Since the power for the stretching is power whose torque is increased during deceleration by the first transmission mechanism T1, even when a large load is applied to the electric prosthetic leg 1 when moving the electric prosthetic leg 1 forward to ascend stairs, the knee joint mechanism 130 can also be reliably stretched from the bending state.

On the other hand, in order to smoothly ascend stairs, as shown in (D) to (G) of FIG. 17, it is necessary to bend the knee joint mechanism 130 from the stretched state while a load is applied to the healthy leg. At this time, it is necessary to quickly fold the electric prosthetic leg 1 and then quickly land the electric prosthetic leg 1 on a next stair. When the knee joint mechanism 130 is bent from the stretched state, large power is not required, but quick action is required.

Specifically, during the transition from the stance to the initial swing ((D) to (E) of FIG. 17), the motor control unit 13 drives the servo motor 242 to cause the first connection and disconnection mechanism 210 and the second connection and disconnection mechanism 220 to transition from the high-torque-side connection state to the high-speed-rotation-side connection state (a transition phase (from the stance state to the initial swing) in FIG. 20), and during this time, the motor M is set into the non-driven state. The non-driven state of the motor M means that the motor M is controlled to generate no power or to be stopped. In the initial swing thereafter (the initial swing phase in FIG. 20), the motor M is rotated in a bending direction opposite to the stretching direction in the high-speed-rotation-side connection state ((E) to (G) of FIG. 17). Then, the power of the motor M is transmitted to the first shaft 181, the second drive gear 185, the second driven gear 186, the connection and disconnection unit 222 of the second connection and disconnection mechanism 220, the second shaft 182, and the spindle unit SP. Accordingly, the sleeve 174 is contracted to approach the transmission T, and the below-knee member 110 to which the transmission T is attached is rotated about the coupling axis 135 with respect to the above-knee member 120 to which the sleeve 174 is coupled, and the knee joint mechanism 130 is bent.

During the transition from the initial swing to the terminal swing ((G) of FIG. 17), the motor M is once set into the non-driven state (a transition phase in FIG. 20 (from the initial swing to the terminal swing)). Then, during the terminal swing ((G) to (H) of FIG. 17), the motor M is rotated in the stretching direction (the terminal swing phase in FIG. 20) in the high-speed-rotation-side connection state. Then, the power of the motor M is transmitted to the first shaft 181, the first drive gear 183, the first driven gear 184, the connection and disconnection unit 222 of the first connection and disconnection mechanism 210, the second shaft 182, and the spindle unit SP. Accordingly, the sleeve 174 is expanded to be separated from the transmission T, and the above-knee member 120 to which the sleeve 174 is coupled is rotated about the coupling axis 135, and the knee joint mechanism 130 is stretched. Since the power for the bending and the stretching during the swing is power whose torque is reduced during acceleration by the second transmission mechanism T2, the knee joint mechanism 130 can be quickly bent and stretched.

During the transition from the terminal swing to the stance ((H) in FIG. 17), the motor control unit 13 drives the servo motor 242 to cause the first connection and disconnection mechanism 210 and the second connection and disconnection mechanism 220 to transition from the high-speed-rotation-side connection state to the high-torque-side connection state (a transition phase (from the terminal swing to the stance) in FIG. 20), and sets the motor M into the non-driven state.

In the stair ascending mode, when the motor control unit 13 controls the motor M, the control (hereinafter, referred to as torque control) is performed based on a torque target value which is a target value of a torque related to a torque for the expansion-contraction device 140 to enlarge or reduce the formed angle during the stance phase, in other words, in the high-torque-side connection state. On the other hand, the motor control unit 13 performs control (hereinafter, referred to as position control) based on a position target value which is a target value of a position related to the formed angle enlarged or reduced by the expansion-contraction device 140 during the swing phase, in other words, in the high-speed-rotation-side connection state. Hereinafter, the torque control, the position control, and a transition between the torque control and the position control will be described in detail.

(Torque Control)

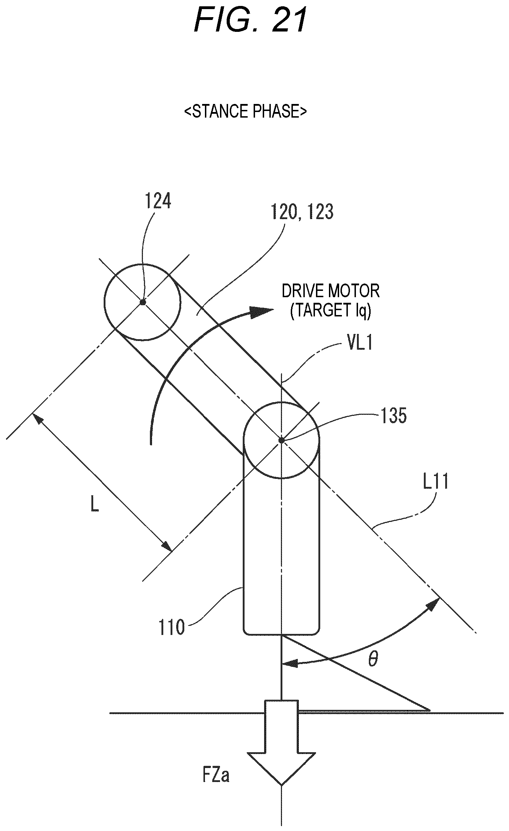

In the torque control, a q-axis current that is in a proportional relationship with the torque for the expansion-contraction device 140 to enlarge or reduce the formed angle is set as a control target. In other words, the torque target value is replaced with a target q-axis current. That is, when the torque target value is determined, the target q-axis current is determined, and when the target q-axis current is determined, the torque target value is determined. Referring to FIG. 21, when the target q-axis current is set as a target Iq [A], a vertical-direction component of a load is set as FZa [N], a thigh length which is a length between the hip joint 124 and the coupling axis 135 is set as L [m], the knee angle is set as θ [deg], and a correction coefficient is set as α, the target Iq is expressed by the following Formula (1).

$$\text{Target Iq [A]} = \text{FZa [N]} \times \text{Sin}\theta \text{ [deg]} \times \text{L [m]} \times \alpha \quad (1)$$

In Formula (1), the below-knee member 110 is regarded as a bar fixed to the above-knee member 120, and a torque is calculated by multiplying a force (FZa × sin θ) required to push up the above-knee member 120 by the thigh length L, so as to calculate the target q-axis current. The motor control unit 13 causes the motor M to generate power in a direction such that the knee joint mechanism 130 in a bent state is stretched, based on the torque target value (target q-axis current). The correction coefficient α is a value based on a torque constant (Kt) [Nm/A] with respect to the q-axis current, and is a value taking into account an adjustment of a stair ascending speed or the like at the time of attachment.

That is, the torque target value (target q-axis current) is determined based on the load FZa (weight), the knee angle θ, and the thigh length L. As also shown in FIG. 20, the motor control unit 13 drives the motor M in the stretching direction based on the torque target value (target q-axis current) in the stance phase of the stair ascending mode in which the torque control is performed.

Instead of the load FZa in Formula (1), the target q-axis current may be set based on a body weight of the user. For example, an absolute value of the body weight may be used, or a body weight multiplied by a coefficient may be used.

(Position Control)

In the position control, a position target value (target angle) is set as a control target. A determination method for the position target value (target angle) is different between the initial swing phase and the terminal swing phase shown in FIG. 20. In other words, the control unit 10 controls the motor M based on the position target value determined based on different parameters between the initial swing phase and the terminal swing phase. In the initial swing phase, two methods are conceivable. Any method may be adopted.

(initial swing Phase_Method 1)

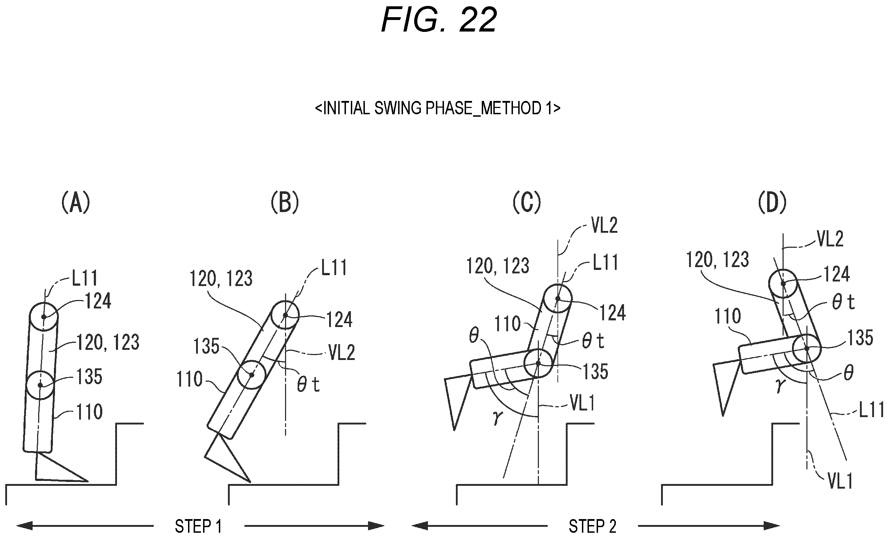

An initial swing phase_method 1 requires an action of swinging the thigh 123 rearward once in the initial swing phase. The method 1 is intended to reliably prevent stumbling over a step that is climbing. (A) and (B) of FIG. 22 show a first step (step 1) of the method 1, and (C) and (D) of FIG. 22 show a second step (step 2) of the method 1. The first step (step 1) is performed in a period from immediately after the transition to the swing to when the electric prosthetic leg 1 is swung rearward such that the thigh angle θt is a predetermined angle (for example, 20 [deg]) or more. The second step (step 2) is performed in a period during which the electric prosthetic leg 1 is swung forward to an ascending stair, and is also a period during which a knee bending angle γ [deg] is fixed and held. As shown in (C) of FIG. 22, the knee bending angle γ [deg] is a formed angle formed by a center line extending in the extending direction of the below-knee member 110 and the vertical line VL1 passing through the coupling axis 135.

In the method 1, the knee angle θ is a control target. A target knee angle θ is expressed by Formula (2).

$$\text{Target Knee Angle } \theta \text{ [deg]} = 0 \text{ [deg]} \quad (2)$$

That is, in the first step (step 1) of the method 1, the target knee angle θ is controlled based on the knee angle θ (θ = 0), and the motor control unit 13 controls the knee joint mechanism 130 to maintain the stretched state. The transition from (A) to (B) of FIG. 22 is realized by the user pivoting the thigh 123 about the hip joint 124 which is a pivoting axis (reference of rotation) of an upper body and the thigh 123.

In the second step (step 2) of the method 1, the knee angle θ is a control target. The target knee angle θ is expressed by Formula (3).

$$\text{Target Knee Angle } \theta \text{ [deg]} = \gamma \text{ [deg]} - \theta t \text{ [deg]} \quad (3)$$

That is, in the second step (step 2) of the method 1, the target knee angle $\theta$ is determined based on the thigh angle $\theta t$ and the knee bending angle $\gamma$, more specifically, based on a difference between the thigh angle $\theta t$ and the knee bending angle $\gamma$, and the motor control unit 13 controls to adjust the knee angle $\theta$ according to a change in the thigh angle $\theta t$ with respect to the knee bending angle $\gamma$ [deg] which is a fixed value. From a viewpoint of preventing stumbling, $\gamma$ [deg] is set to, for example, about 80 [deg].

(Initial Swing Phase _Method 2)

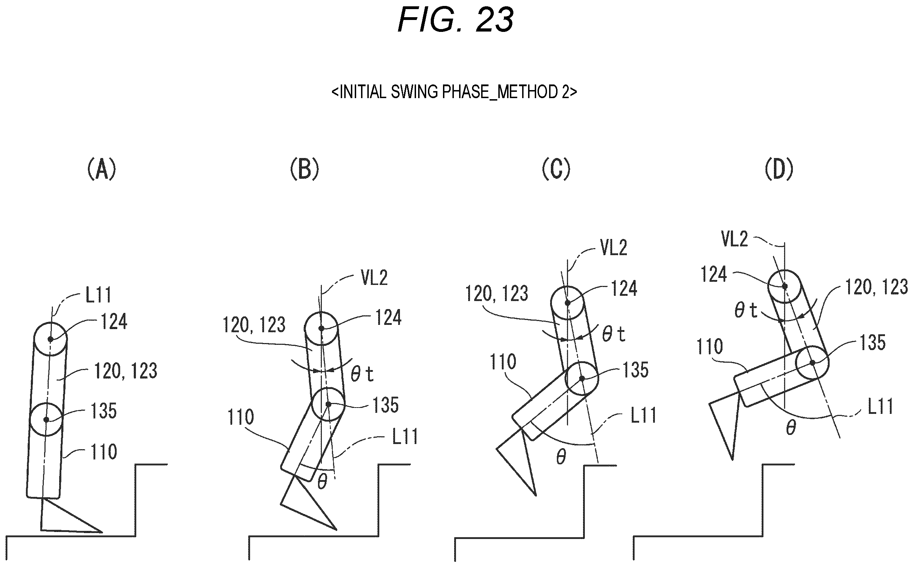

An initial swing phase_method 2 is intended to eliminate the action of swinging the thigh 123 rearward once performed in the method 1, to shorten a walking time, and to reduce a burden on the user when ascending stairs. In the method 2, the determination method for the control target is unified through (A) to (D) of FIG. 23. In other words, the control unit 10 controls the motor M based on the position target value determined based on the same parameter through the initial swing phase.

In the method 2, the knee angle $\theta$ is a control target. The target knee angle $\theta$ is expressed by Formula (4) in which a magnification is set as $\delta$ and a correction angle is set as $\varepsilon$ [deg].

$$\text{Target Knee Angle } \theta \text{ [deg]} = \delta \times (\theta t \text{ [deg]} - \varepsilon \text{ [deg]}) \quad (4)$$

That is, in the method 2, the target knee angle $\theta$ is determined based on the thigh angle $\theta t$, and the motor control unit 13 controls the knee angle $\theta$ based on the thigh angle $\theta t$ such that the below-knee member 110 is bent rearward with a certain magnification. As the thigh 123 swings forward, it is necessary to increase the knee bending angle $\gamma$ in order to prevent stumbling. That is, it is necessary to increase the knee bending angle $\gamma$ in proportion to the thigh angle $\theta t$. From the viewpoint of preventing stumbling, the magnification $\delta$ is set to, for example, 3, and $\varepsilon$ is set to, for example, about 10 [deg].

(Terminal Swing Phase)

The terminal swing phase is a ground contact preparation period. In the terminal swing phase, the knee angle $\theta$ is a control target. The target knee angle $\theta$ is expressed by Formula (5).

$$\text{Target Knee Angle } \theta \text{ [deg]} = \text{Thigh Angle } \theta t \text{ [deg]} + \text{Adjustment Angle } \beta \text{ [deg]} \quad (5)$$

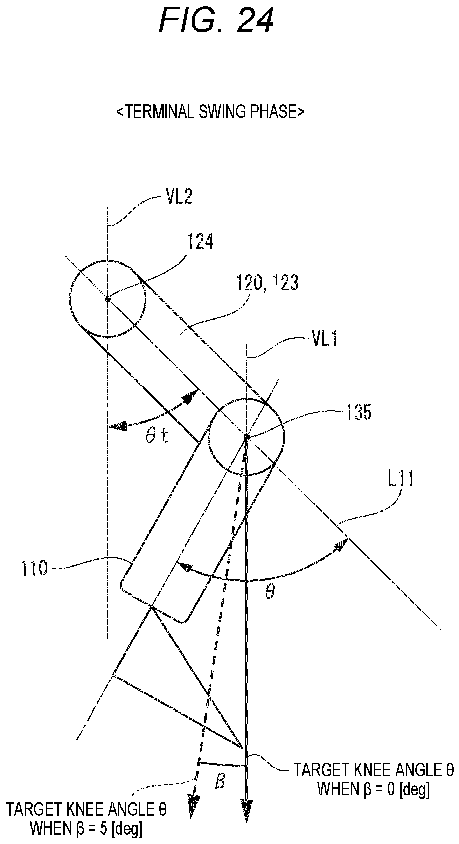

That is, the target knee angle $\theta$ is determined based on the thigh angle $\theta t$ and the adjustment angle $\beta$, and as shown in FIG. 24, the motor control unit 13 controls the below-knee members 110 to be normally substantially perpendicular to a horizontal floor surface regardless of the thigh angle $\theta t$ for body weight support. In other words, the knee angle $\theta$ is controlled to be substantially equal to the thigh angle $\theta t$. As shown in FIG. 24, the adjustment angle $\beta$ [deg] is a formed angle formed by the center line extending in the extending direction of the below-knee member 110 and the vertical line VL1 passing through the coupling axis 135, the adjustment angle $\beta$ takes a negative value when the below-knee member 110 is in front of the vertical line VL1, and the adjustment angle $\beta$ takes a positive value when the below-knee member 110 is behind the vertical line VL1. The adjustment angle $\beta$ [deg] is an adjustment angle set in consideration of, for example, a feeling of the user when ascending stairs. The adjustment angle $\beta$ is set such that the below-knee member 110 is positioned on the same side as the above-knee member 120 with respect to the vertical line VL1. In the electric prosthetic leg 1, the adjustment angle $\beta$ is set such that a toe is set to be lower than a heel, and is, for example, 5 [deg].

(Transition Conditions)

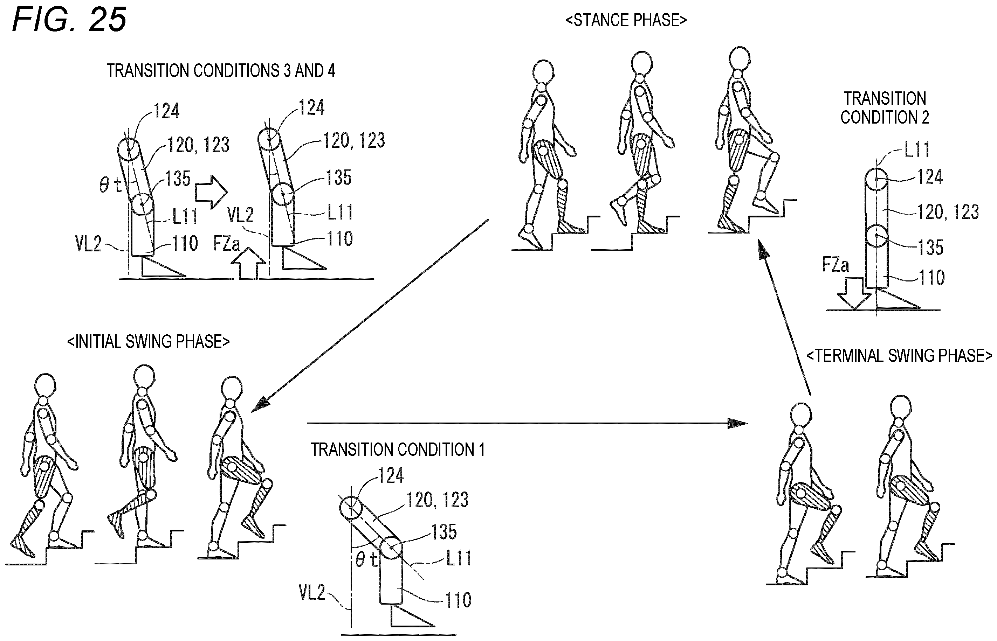

When ascending stairs, actions of the initial swing phase, the terminal swing phase, and the stance phase are repeated. Next, transition conditions will be described with reference to FIG. 25.

(Transition from Initial Swing Phase to Terminal Swing Phase)

The transition from the initial swing phase to the terminal swing phase is executed when the following transition condition 1 is satisfied.

Transition condition 1: the thigh angle θt falls within a first predetermined range.

The first predetermined range is, for example, a range less than -50 [deg]. That is, since the thigh angle θt takes a negative value when the above-knee member 120 is in front of the vertical line VL2 (see (B) of FIG. 19) as described above, it is determined that the thigh 123 (the above-knee member 120) is swung up forward by a predetermined amount, under the condition.

(Transition from Terminal Swing Phase to Stance Phase)

The transition from the terminal swing phase to the stance phase is executed when the following transition condition 2 is satisfied.

ransition condition 2: the load FZa falls within a second predetermined range.

The second predetermined range is, for example, a range of -110 [N] or less. That is, since the load sensor 272 is provided such that the tensile load takes a positive value and the compression load takes a negative value as described above, when the load FZa is equal to or less than -110 [N], it is determined that the electric prosthetic leg 1 is in contact with the ground and transitions to the stance state. This is coincident with a determination criterion of the phase determination unit 11 for the transition from the swing phase to the stance phase.

(Transition from Stance Phase to Initial Swing Phase)

The transition from the stance phase to the initial swing phase is executed when the following transition condition 3 is satisfied.

Transition condition 3: The load FZa falls within a third predetermined range.

The third predetermined range is, for example, a range of -90 [N] or more. This is coincident with a determination criterion of the phase determination unit 11 for the transition from the stance phase to the swing phase.
When the transition condition 3 is satisfied after the following transition condition 4 is satisfied, it is preferable to determine that the electric prosthetic leg 1 leaves the ground and transitions to a swing state.

Transition condition 4: the thigh angle θt falls within a fourth predetermined range.

The fourth predetermined range is, for example, a range less than -15 [deg]. By determining under the transition condition 4 that the thigh 123 (the above-knee member 120) is moved rearward as the body weight moves, and then determining under the third transition condition that the load FZa decreases, the transition from the swing phase to the stance phase can be more reliably determined.

<Flat Ground and Stair Descending Mode>

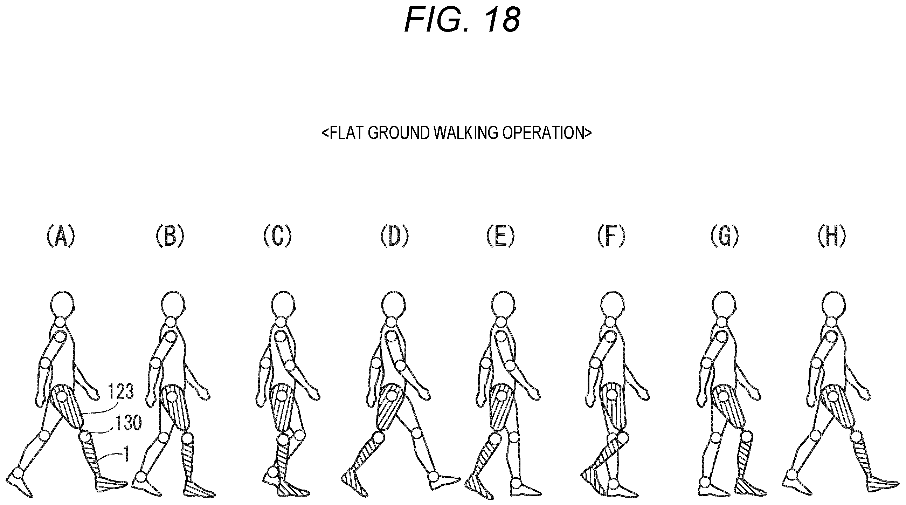

A mode in which the user walks to advance on a flat ground or walks to ascend stairs is the flat ground and stair descending mode. The flat ground means that there is no step such as a stair, and is a concept including an uphill slope and a downhill slope in addition to a horizontal place. FIG. 18 is a diagram showing actions (flat ground walking actions) of the human and the electric prosthetic leg when walking on the flat ground.
(A) to (D) of FIG. 18 are diagrams showing the stance phase, (D) of FIG. 18 is a diagram showing the transition phase from the stance to the swing, (E) to (H) of FIG. 18 are diagrams showing the swing phase, and (H) of FIG. 18 is a diagram showing the transition phase from the swing to the stance.
In the flat ground and stair descending mode, the motor M is normally in the non-driven state. In the non-driven state of the motor M, as shown in (A) to (D) of FIG. 18, it is necessary to prevent so-called giving way in the stance phase in which a load is applied to the electric prosthetic leg 1.
During the stance ((A) to (D) of FIG. 18), the motor control unit 13 drives the servo motor 242 to set the first connection and disconnection mechanism 210 and the second connection and disconnection mechanism 220 into the high-speed-rotation-side connection state. (Stance Phase of FIG. 20). In the high-speed-rotation-side connection state, since the connection and disconnection unit 222 is in the on state, the motor M and the spindle unit SP are in the power transmission state via the second transmission mechanism T2. In the high-speed-rotation-side connection state, when the motor M is held in the non-driven state, an external force in the bending direction acting on the electric prosthetic leg 1 is transmitted from the spindle unit SP to the motor M via the second transmission mechanism T2, frictions of the motor M and the transmission T are utilized to dampen the external force in the bending direction, thus preventing the so-called giving way. The reason why the high-speed-rotation-side connection state is set during the stance phase in the flat ground and stair descending mode is to prevent the so-called giving way, and the high-torque-side connection state may be set without necessarily requiring the high-speed-rotation-side connection state. In FIG. 20 and the following description, a case will be described in which the first connection and disconnection mechanism 210 and the second connection and disconnection mechanism 220 are set to the high-speed-rotation-side connection state during the stance phase in the flat ground and stair descending mode.

On the other hand, as shown in (E) to (H) of FIG. 18, it is necessary to freely swing the electric prosthetic leg 1 during the swing in which no load from the outside is applied to the electric prosthetic leg 1. Therefore, during the transition from the stance to the swing ((D) of FIG. 18), the motor control unit 13 drives the servo motor 242 to set the first connection and disconnection mechanism 210 and the second connection and disconnection mechanism 220 into the disconnection state (transition phase (from the stance to the swing) in FIG. 20). In the disconnection state, since the connection and disconnection unit 212 and the connection and disconnection unit 222 are in the off state, the motor M and the spindle unit SP are in the disconnection state of being not connected with each other, during the swing (the swing phase in FIG. 20). In this state, when the motor M is held in the non-driven state, free stretching and bending of the knee joint mechanism 130 are allowed. Descending stairs is controlled in the same way as walking on flat ground.

In the transition phase from the swing to the stance ((H) in FIG. 18), the motor control unit 13 drives the servo motor 242 to cause the first connection and disconnection mechanism 210 and the second connection and disconnection mechanism 220 to transition from the disconnection state to the high-speed-rotation-side connection state (the transition phase (from the swing to the stance) in FIG. 20).

Here, a function of preventing the giving way will be described in more detail.

As described above, during the stance in the flat ground and stair descending mode, it is necessary to support the body weight of the user with the electric prosthetic leg 1 to prevent the giving way, but the body weight of the user is not constant. Therefore, it is preferable that the frictions generated in the motor M and the transmission T are variable.

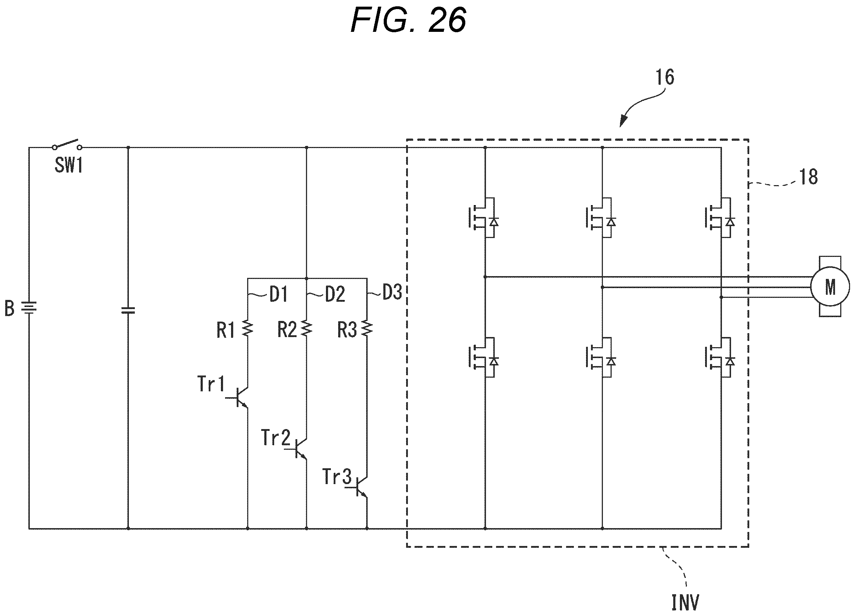

First, a method of changing the friction generated in the motor M will be described with reference to FIGs. 26 to 28. FIG. 26 is a diagram showing an example of an electric circuit 16 connecting the battery B and the motor M. In the electric circuit 16, a path connecting the battery B and the servo motor 242 is omitted. An inverter 18 is provided between the battery B and the motor M via a switch SW1. The inverter 18 converts DC power from the battery B into AC power and supplies the AC power to the motor M. Between the switch SW1 and the inverter 18, a circuit D1 in which a resistor R1 and a transistor Tr1 are connected in series, a circuit D2 in which a resistor R2 and a transistor Tr2 are connected in series, and a circuit D3 in which a resistor R3 and a transistor Tr3 are connected in series are connected in parallel. In the electric circuit 16, by setting the switch SW1 to an open state (OFF) and setting at least one of the transistors Tr1 to Tr3 to a closed state (ON), a power generation brake can be generated and the friction (braking force) with respect to the rotation of the motor M can be generated.

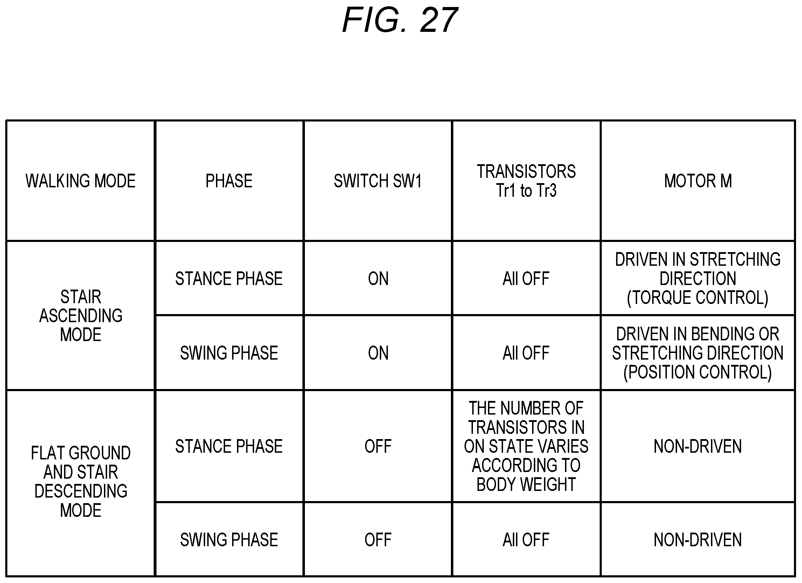

Referring also to FIG. 27, in such an electric circuit 16, during the stance phase and the swing phase in the stair ascending mode, that is, when the motor M is driven, the switch SW1 is in a closed state (ON) and all the transistors Tr1 to Tr3 are in an open state (OFF) to control the switching of the inverter 18, and thus the motor M can be rotated in the stretching direction or the bending direction. On the other hand, in the flat ground and stair descending mode, that is, when the motor M is not driven, the switch SW1 is in an open state (OFF) to stop the power supply from the battery B to the motor M.

During the swing phase in the flat ground and stair descending mode, all the transistors Tr1 to Tr3 are in an open state (OFF). During the swing phase in the flat ground and stair descending mode, since free stretching and bending of the knee joint mechanism 130 are allowed as described above, no braking force is generated by the power generation brake. On the other hand, during the stance phase in the flat ground and stair descending mode, at least one of the transistors Tr1 to Tr3 is set to a closed state (ON), and thus the friction can be generated by the power generation brake. The friction based on the power generation brake depends on the number of transistors that are in a closed state (ON), and the friction based on the power generation brake increases as the number of transistors that are in a closed state increases.

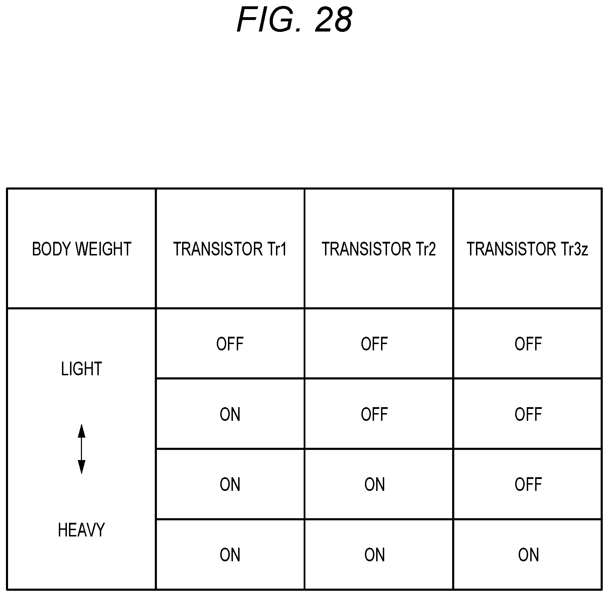

Therefore, as shown in FIG. 28, as the body weight of the user increases, the number of transistors to be set into a closed state (ON) during the stance phase in the flat ground and stair descending mode is increased. That is, when the body weight of the user is large, the number of transistors to be set to a close state (ON) is reduced, and when the body weight of the user is small, the number of transistors to be set to the close state (ON) is increased. This setting is preferably set as an initial setting in a program when the user of the electric prosthetic leg 1 is determined.

Further, a method of increasing the friction generated in the motor M is not limited to changing the number of transistors to be set to a closed state (ON). For example, only one transistor (only the circuit D1) may be used, and a duty ratio of pulse width modulation (PWM) control when the transistor is in a closed state (ON) may be changed. For example, the duty ratio of the PWM control when the transistor is in a closed state (ON) may be set to be small when the body weight of the user is small, and the duty ratio of the PWM control when the transistor is in a closed state (ON) may be set to be large when the body weight of the user is large. The setting is also preferably set as an initial setting in a program when the user of the electric prosthetic leg 1 is determined.

Next, a method of changing the friction of the transmission T will be described.

In order to increase the friction of the transmission T, a rotation speed of the motor M when the external force in the bending direction acting on the electric prosthetic leg 1 is transmitted from the spindle unit SP to the motor M via the second transmission mechanism T2 may be increased. Therefore, during the stance phase in the flat ground and stair descending mode, the rotation speed of the motor M in the high-speed-rotation-side connection state is set to be increased. That is, the transmission ratio in the high-speed-rotation-side connection state, that is, the second transmission ratio of the second transmission mechanism T2 is increased. Since a ratio of the second transmission ratio with respect to the first transmission ratio is preferably in a range of 1.5 to 3.0, it is preferable to adjust the first transmission ratio of the first transmission mechanism T1 by an amount by which the second transmission ratio of the second transmission mechanism T2 is increased. Therefore, the second transmission ratio of the second transmission mechanism T2 is set to be increased when the body weight of the user is large, and the second transmission ratio of the second transmission mechanism T2 is set to be decreased when the body weight of the user is small. During the stance phase in the flat ground and stair descending mode, in a case where the high-torque-side connection state is set instead of the high-speed-rotation-side connection state, the first transmission ratio of the first transmission mechanism T1 is set to be increased in order to increase the friction of the transmission T. Regarding the setting, it is also preferable that specifications of the transmission T are set when the user of the electric prosthetic leg 1 is determined.

FIG. 20 is a table summarizing phases and corresponding control methods in the stair ascending mode and the flat ground and stair descending mode. As shown in FIG. 20, the motor control unit 13 performs the torque control on the motor M during the stance phase in the stair ascending mode, and performs the position control on the motor M during the swing phase in the stair ascending mode. In this way, even in the same stair ascending mode, the control on the motor M varies between the stance phase and the swing phase, and thus the electric prosthetic leg 1 can be appropriately controlled.

Further, even in the same stance phase, the motor control unit 13 performs the torque control on the motor M in the stair ascending mode, and does not drive the motor M, that is, performs control such that the power of the motor M is not generated or the motor M is stopped in the flat ground and stair descending mode. In this way, even during the stance phase, the control on the motor M varies in the flat ground and stair descending mode and in the stair ascending mode, and thus the electric prosthetic leg 1 can be appropriately controlled according to the walking mode.

Further, even in the same swing phase, the motor control unit 13 performs the position control on the motor M in the stair ascending mode, and does not drive the motor M, that is, performs control such that the power of the motor M is not generated or the motor M is stopped in the flat ground and stair descending mode. In this way, even during the swing phase, the control on the motor M varies in the flat ground and stair descending mode and in the stair ascending mode, and thus the electric prosthetic leg 1 can be appropriately controlled according to the walking mode.

The control method in the stair ascending mode and the flat ground and stair descending mode can be realized by executing a program prepared in advance by a computer (processor). The program is stored in a storage medium readable by a computer, and is executed by being read from the storage medium. In addition, the program may be provided in a form stored in a non-transitory storage medium such as a flash memory, or may be provided via a network such as the Internet.

Although the various embodiments have been described above with reference to the drawings, it is needless to say that the present invention is not limited to these examples. It is apparent that those skilled in the art can conceive of various modifications and changes within the scope described in the claims, and it is understood that such modifications and changes naturally fall within the technical scope of the present invention. In addition, constituent elements in the embodiment described above may be freely combined without departing from the gist of the present invention.

For example, in the above embodiment, the transmission T has two power transmission paths of the first transmission mechanism T1 and the second transmission mechanism T2, but is not limited to thereto, and may include only one transmission mechanism and performs speed change at the same transmission ratio during the stance phase and the swing phase in the stair ascending mode.

For example, in the above embodiments, although prosthetic leg devices (electric prosthetic leg) applied to a knee joint as embodiments of the joint device of the present invention are described, the present invention is not limited thereto, and may be a prosthetic limb device (electric prosthetic limb) applied to an elbow joint, and the wearer may be an animal other than a human, or a robot. When applied to an elbow joint, the below-knee member 110 in the above embodiment is situated at a distal end side of a wearer with respect to the above-knee member 120, that is, a forearm.

In the present description, at least the following matters are described. In the parentheses, the corresponding constituent elements and the like in the above embodiment are shown, but the present invention is not limited thereto.

　　　(1) A joint device (electric prosthetic leg 1), including:

a first member (below-knee member 110);

a second member (above-knee member 120);

a coupling portion (knee joint mechanism 130) configure to couple the first member and the second member such that a formed angle (second formed angle θ2, knee angle θ) formed between the first member and the second member is variable; and

an enlarging and reducing device (enlarging and reducing device 200) capable of enlarging and reducing the formed angle formed between the first member and the second member, in which

the enlarging and reducing device includes a power source (motor M), a power transmission unit (transmission T) configured to transmit power of the power source, and a control unit (control unit 10) configured to control the power source,

the joint device is provided to transition between a weighted state (stance phase) where a weight applied from an outside is received and a non-weighted state (swing phase) where no weight is received, and

the control unit,

(A) when the joint device is in the weighted state,

controls (torque control) the power source based on a torque target value which is a target value of a torque related to a torque for the enlarging and reducing device to enlarge or reduce the formed angle, and

(B) when the joint device is in the non-weighted state,

controls (position control) the power source based on a position target value which is a target value of a position related to the formed angle enlarged or reduced by the enlarging and reducing device.

According to (1), the control on the power source varies in the weighted state and in the non-weighted state, and thus the joint device can be appropriately controlled.

(2) The joint device according to (1), in which

the torque target value is determined based on the weight applied (load FZa).

According to (2), the torque target value can be appropriately set.

(3) The joint device according to (1), in which

the torque target value is determined based on a weight (body weight) of a wearer.

According to (3), the torque target value can be appropriately set.

(4) The joint device according to any one of (1) to (3), in which

the torque target value is determined based on the formed angle or based on a supplementary angle (knee angle θ, second formed angle θ2) of the formed angle.

According to (4), the torque target value can be appropriately set.

(5) The joint device according to any one of (1) to (4), in which

the joint device is attached on a first portion (thigh portion 123) of a wearer among the first portion and a second portion (upper body) that pivots relative to the first portion,

the second member is provided to be attached to the first portion such that the first member is situated at a distal end side of the wearer than the second member, and

the torque target value is determined based on a length (thigh length L) between the coupling portion and another coupling portion (hip joint 124) of the wearer, the another coupling portion coupling the first portion and the second portion such that another formed angle formed between the first portion and the second portion is variable.

According to (5), the torque target value can be appropriately set.

(6) The joint device according to any one of (1) to (5), in which

the control unit,

(A) when the joint device is in the weighted state,

controls the power source to generate power in a direction such that the joint device in a state where the first member and the second member are bent becomes a state where the first member and the second member are stretched.

According to (6), the joint device can be stretched while supporting a load.

(7) The joint device according to any one of (1) to (6), in which

the position target value is determined based on a first formed angle (thigh angle θt) between the second member and a reference line (vertical line VL2) passing through a pivoting axis (hip joint 124) of a first portion (thigh 123) of a wearer and a second portion (upper body) that pivots relative to the first portion.

According to (7), the position target value can be appropriately set.

(8) The joint device according to any one of (1) to (7), in which

the position target value is determined based on a second formed angle (adjustment angle β) between the first member and another reference line (vertical line VL1) passing through another pivoting axis (coupling axis 135) of the first member and the second member.

According to (8), the position target value can be appropriately set.

(9) The joint device according to (8), in which

the second formed angle is determined such that the first member is positioned on a same side as the second member with respect to the another reference line.

According to (9), the position target value can be set more appropriately.

(10) The joint device according to (8) which is dependent on (7), in which

> when a period from a transition from the weighted state to the non-weighted state to a transition to the weighted state again is defined as a non-weighted period, and
> a latter half of the non-weighted period is defined as a latter non-weighted period (terminal swing phase),
> the position target value is determined based on the first formed angle or the second formed angle when the joint device is in the latter non-weighted period.

According to (10), the position target value can be set more appropriately.

(11) The joint device according to any one of (1) to (10), in which

the position target value is determined based on a third formed angle (knee bending angle γ) between the first member and another reference line (vertical line VL1) passing through another pivoting axis (coupling axis 135) of the first member and the second member.

According to (11), the position target value can be set more appropriately.

(12) The joint device according to (11), in which

the position target value is determined based on a difference between the third formed angle and a first formed angle (thigh angle θt) between the second member and a reference line (vertical line VL2) passing through a pivoting axis (hip joint 124) of a first portion (thigh 123) of a wearer and a second portion (upper body) that pivots relative to the first portion.

According to (12), the position target value can be set more appropriately.

(13) The joint device according to (11) or (12), in which

> when a period from a transition from the weighted state to the non-weighted state to a transition to the weighted state again is defined as a non-weighted period, and
> an earlier half of the non-weighted period is defined as an earlier non-weighted period (initial swing phase),
> the position target value is determined based on the third formed angle when the joint device is in the earlier non-weighted period.

According to (13), the position target value can be set more appropriately.

(14) The joint device according to any one of (1) to (10), in which

> when a period from a transition from the weighted state to the non-weighted state to a transition to the weighted state again is defined as a non-weighted period, and
> an earlier half of the non-weighted period is defined as an earlier non-weighted period (initial swing phase), and a latter half is defined as a latter non-weighted period (terminal swing phase),
> the control unit controls the power source based on the position target value determined by a determination method which is different between the earlier non-weighted period and the latter non-weighted period.

According to (14), each of an operation during the earlier non-weighted period and an operation during the latter non-weighted period can be optimized.

(15) The joint device according to (14), in which

the control unit switches to control for the latter non-weighted period, when a first formed angle (thigh angle θt) between the second member and a reference line (vertical line VL2) passing through a pivoting axis (hip joint 124) of a first portion of a wearer and a second portion that pivots relative to the first portion falls within a first predetermined range (less than -50 [deg]) during the earlier non-weighted period.

According to (15), the control can be smoothly shifted from that for the earlier non-weighted period to that for the latter non-weighted period.

(16) The joint device according to (14) or (15), in which

> when a period from a transition from the non-weighted state to the weighted state to a transition to the non-weighted state again is defined as a weighted period,

the control unit switches to control for the weighted period when the applied weight falls within a second predetermined range (-110 [N] or less) during the latter non-weighted period.

According to (16), the control can be smoothly shifted from that for the latter non-weighted period to that for the weighted period.

(17) The joint device according to any one of (1) to (16), in which

when a period from a transition from the weighted state to the non-weighted state to a transition to the weighted state again is defined as a non-weighted period, and

a period from a transition from the non-weighted state to the weighted state to a transition to the non-weighted state again is defined as a weighted period,

the control unit switches to control for the non-weighted period when the applied weight falls within a third predetermined range (-90 [N] or more) during the weighted period.

According to (17), the control can be smoothly shifted from that for the weighted period to that for the non-weighted period.

(18) The joint device according to (17), in which

the control unit switches to control for the non-weighted period, when the applied weight falls within the third predetermined range after a first formed angle (thigh angle θt) between the second member and a reference line (vertical line VL2) passing through a pivoting axis (hip joint 124) of a first portion of a wearer and a second portion that pivots relative to the first portion falls within a fourth predetermined range (less than -15 [deg]) during the weighted period.

According to (18), the transition from the weighted period to the non-weighted period can be more appropriately determined.

(19) The joint device according to (17) or (18), in which

the control unit switches to control for the weighted period when the applied weight falls within a fifth predetermined range (-110 [N] or less) during the non-weighted period.

According to (19), the control can be smoothly shifted from that for the non-weighted period to that for the weighted period.

(20) The joint device according to any one of (1) to (19), in which

the enlarging and reducing device further includes a connection and disconnection mechanism disposed on a power transmission path of the power transmission unit and configured to switch between connection and disconnection of power in the power transmission path,

the control unit further controls the connection and disconnection mechanism, and

the control unit,

(B) when the joint device is in the non-weighted state,

controls the connection and disconnection mechanism to a disconnection state where the power transmission path is disconnected (swing phase in a flat ground and stair descending mode), and

controls the power source such that the power source does not generate power or is stopped, instead of controlling the power source based on the position target value.

According to (20), the joint device can be appropriately controlled in both the weighted state and the non-weighted state.

(21) The joint device according to (20), in which

the control unit,

(A) when the joint device is in the weighted state,

controls the connection and disconnection mechanism to a connection state where the power transmission path is connected (stance phase in the flat ground and stair descending mode), and

controls the power source such that the power source does not generate power or is stopped, instead of controlling the power source based on the torque target value.

According to (21), the joint device can be appropriately controlled in both the weighted state and the non-weighted state.

(22) The joint device according to (20) or (21), in which

the power source is a permanent-magnet-type electric motor.

According to (22), a friction generated when the power source is not driven can be utilized.

(23) The joint device according to any one of (20) to (22), in which
the control unit is configured to be capable of adjusting a braking force for stopping the power source.
According to (23), the braking force utilizing the friction generated when the power source is not driven can be adjusted.
(24) The joint device according to (23), in which
the control unit causes to generate the braking force set according to a weight (body weight) of a wearer.
According to (24), the braking force corresponding to the body weight can be generated.
(25) The joint device according to any one of (1) to (24), in which

the power transmission unit has:

a first power transmission path (first transmission mechanism T1) for transmitting the power at a first transmission ratio; and
a second power transmission path (second transmission mechanism T2) for transmitting the power at a second transmission ratio different from the first transmission ratio,

the enlarging and reducing device further includes:

a first connection and disconnection mechanism (first connection and disconnection mechanism 210) disposed on the first power transmission path and configured to switch between connection and disconnection of power in the first power transmission path; and
a second connection and disconnection mechanism (second connection and disconnection mechanism 220) disposed on the second power transmission path and configured to switch between connection and disconnection of power in the second power transmission path, and

the control unit further controls the first connection and disconnection mechanism and the second connection and disconnection mechanism.

According to (25), the control unit controls the first connection and disconnection mechanism and the second connection and disconnection mechanism in addition to the power source.
(26) The joint device according to (25), in which

when the first transmission ratio is defined as a ratio of a post-transmission rotation speed with respect to a pre-transmission rotation speed which is a rotation speed on a power source side in the first power transmission path relative to a first transmission unit (first transmission mechanism T1), and
the second transmission ratio is defined as a ratio of a post-transmission rotation speed with respect to a pre-transmission rotation speed which is a rotation speed on the power source side in the second power transmission path relative to a second transmission unit (second transmission mechanism T2),
the first transmission ratio is configured to be smaller than the second transmission ratio.

According to (26), the power of the power source can be transmitted at different transmission ratios.
(27) The joint device according to (25) or (26), in which
at least one of the first transmission ratio and the second transmission ratio is determined based on a weight (body weight) of a wearer.
According to (27), the transmission ratio corresponding to the body weight can be set.
(28) The joint device according to any one of (25) to (27), in which
a ratio between the first transmission ratio and the second transmission ratio is set to 1.5 to 3.0.
According to (28), a high torque state and a high-speed rotation state can be appropriately balanced.
(29) The joint device according to any one of (1) to (28), in which
the joint device is a prosthetic limb device which is attached to a wearer such that the first member is situated at a distal end side of the wearer than the second member, and the coupling portion is provided to function as a joint of the wearer.
According to (29), a smooth bending operation and a smooth stretching operation can be performed.
(30) The joint device according to (29), in which
the prosthetic limb device is a prosthetic leg device to be attached to a leg of the wearer.
According to (30), the smooth bending operation and the smooth stretching operation of a prosthetic leg can be performed.
(31) The joint device according to (30), in which
the prosthetic leg device is provided such that the second member is attached to a thigh (thigh 123) of the leg, and the coupling portion is provided to function as a knee joint between the thigh and a lower leg.

According to (31), the smooth bending operation and the smooth stretching operation can be performed.

(32) A joint device (electric prosthetic leg 1), including:

a first member (below-knee member 110);

a second member (above-knee member 120);

a coupling portion (knee joint mechanism 130) configured to couple the first member and the second member such that a formed angle formed between the first member and the second member is variable; and

an enlarging and reducing device (enlarging and reducing device 200) capable of enlarging and reducing the formed angle formed between the first member and the second member, in which

the enlarging and reducing device includes a power source (motor M), a power transmission unit (transmission T) configured to transmit power of the power source, an connection and disconnection mechanism (first connection and disconnection mechanism 210, second connection and disconnection mechanism 220) disposed on a power transmission path of the power transmission unit and configured to switch between connection and disconnection of power in the power transmission path, and a control unit (control unit 10) configured to control the power source and the connection and disconnection mechanism,

the joint device is provided to transition between a weighted state (stance phase) where a weight applied from an outside is received and a non-weighted state (swing phase) where no weight is received, and

the control unit,

(A) when the joint device is in the weighted state (stance state in a flat ground and stair descending mode),

controls the connection and disconnection mechanism to a connection state where the power transmission path is connected, and

controls the power source such that the power source does not generate power or is stopped, and

(B) when the joint device is in the non-weighted state (swing state in the flat ground and stair descending mode),

controls the connection and disconnection mechanism to a disconnection state where the power transmission path is disconnected, and

controls the power source such that the power source does not generate power or is stopped.

According to (32), the joint device can be appropriately controlled in both the weighted state and the non-weighted state.

(33) The joint device according to (32), in which

the joint device is a prosthetic limb device which is attached to a wearer such that the first member is situated at a distal end side of the wearer than the second member, and the coupling portion is provided to function as a joint of the wearer, the prosthetic limb device is a prosthetic leg device to be attached to a leg of the wearer, and

the control unit,

(A) when the joint device is in the weighted state,

(a) in a case of walking to advance on a flat ground, or in a case of walking to descend stairs (flat ground and stair descending mode),

controls the connection and disconnection mechanism to a connection state where the power transmission path is connected (stance phase), and

controls the power source such that the power source does not generate power or is stopped, and

(b) in a case of walking to ascend stairs (stair ascending mode),

controls the connection and disconnection mechanism to the connection state where the power transmission path is connected (stance phase), and

controls the power source based on a torque target value which is a target value of a torque related to a torque for the enlarging and reducing device to enlarge or reduce the formed angle.

According to (33), even in the same weighted state, the control on the power source varies in the flat ground and stair descending mode and in the stair ascending mode, and thus the joint device can be appropriately controlled.

(34) The joint device according to (32) or (33), in which

the joint device is a prosthetic limb device which is attached to a wearer such that the first member is situated at a distal end side of the wearer than the second member, and the coupling portion is provided to function as a joint of the wearer, the prosthetic limb device is a prosthetic leg device to be attached to a leg of the wearer, and the control unit,

(B) when the joint device is in the non-weighted state,

    (a) in a case of walking to advance on a flat ground, or in a case of walking to descend stairs (flat ground and stair descending mode),

        controls the connection and disconnection mechanism to the disconnection state where the power transmission path is disconnected (swing phase), and
        controls the power source such that the power source does not generate power or is stopped, and

    (b) in a case of walking to ascend stairs (stair ascending mode),

        controls the connection and disconnection mechanism to the connection state where the power transmission path is connected (swing phase), and
        controls the power source based on a position target value which is a target value of a position related to the formed angle enlarged or reduced by the enlarging and reducing device.

According to (34), even in the same non-weighted state, the control on the power source varies in the flat ground and stair descending mode and in the stair ascending mode, and thus the joint device can be appropriately controlled.

(35) A joint device control method for controlling a joint device (electric prosthetic leg 1) including:

a first member (below-knee member 110),
a second member (above-knee member 120),
a coupling portion (knee joint mechanism 130) configured to couple the first member and the second member such that a formed angle (second formed angle $\theta2$, knee angle $\theta$) formed between the first member and the second member is variable, and
an enlarging and reducing device (enlarging and reducing device 200) capable of enlarging and reducing the formed angle formed between the first member and the second member, in which
the enlarging and reducing device including a power source (motor M), and a power transmission unit (transmission T) configured to transmit power of the power source,
the joint device is provided to transition between a weighted state (stance phase) where a weight applied from an outside is received and a non-weighted state (swing phase) where no weight is received, and
the control method includes the steps of:

    (A) when the joint device is in the weighted state (stance phase in a stair ascending mode),
    controlling the power source based on a torque target value which is a target value of a torque related to a torque for the enlarging and reducing device to enlarge or reduce the formed angle; and
    (B) when the joint device is in the non-weighted state (swing phase in the stair ascending mode),
    controlling the power source based on a position target value which is a target value of a position related to the formed angle enlarged or reduced by the enlarging and reducing device.

According to (35), the control on the power source varies in the weighted state and in the non-weighted state, and thus the joint device can be appropriately controlled.

(36) A joint device control program for controlling a joint device (electric prosthetic leg 1) including:

a first member (below-knee member 110),
a second member (above-knee member 120),
a coupling portion (knee joint mechanism 130) configured to couple the first member and the second member such that a formed angle (second formed angle $\theta2$, knee angle $\theta$) formed between the first member and the second member is variable, and
an enlarging and reducing device (enlarging and reducing device 200) capable of enlarging and reducing the formed angle formed between the first member and the second member, in which
the enlarging and reducing device including a power source (motor M), and a power transmission unit (transmission T) configured to transmit power of the power source,
the joint device is provided to transition between a weighted state (stance phase) where a weight applied from an outside is received and a non-weighted state (swing phase) where no weight is received, and
the control program causes a computer to perform the steps of:

(A) when the joint device is in the weighted state,
controlling the power source based on a torque target value which is a target value of a torque related to a torque
for the enlarging and reducing device to enlarge or reduce the formed angle; and
(B) when the joint device is in the non-weighted state,
controlling the power source based on a position target value which is a target value of a position related to the
formed angle enlarged or reduced by the enlarging and reducing device.

According to (36), the control on the power source varies in the weighted state and in the non-weighted state, and
thus the joint device can be appropriately controlled.

(37) A computer-readable storage medium storing the control program according to (36).

According to (37), the control on the power source varies in the weighted state and in the non-weighted state, and
thus the joint device can be appropriately controlled.

The present application is based on a Japanese Patent Application (Japanese Patent Application No.
2022-142463) filed on September 7, 2022, and the contents thereof are incorporated herein by reference.

REFERENCE SIGNS LIST

1: electric prosthetic leg (joint device)
10: control unit
110: below-knee member (first member)
120: above-knee member (second member)
123: thigh (first portion)
124: hip joint (another coupling portion, pivoting axis)
130: knee joint mechanism (coupling portion)
135: coupling axis (another pivoting axis)
200: enlarging and reducing device
210: first connection and disconnection mechanism
220: second connection and disconnection mechanism
FZa: load (weight)
L: thigh length (length between the coupling portion and another coupling portion)
L11: extension Line
M: motor (power source)
T: transmission (power transmission unit)
T1: first transmission mechanism (first power transmission path)
T2: second transmission mechanism (second power transmission path)
VL1: vertical line (another reference line)
VL2: vertical line (reference line)
$\theta$t: thigh angle

**Claims**

1.  A joint device comprising:

a first member;
a second member;
a coupling portion configured to couple the first member and the second member such that a formed angle formed
between the first member and the second member is variable; and
an enlarging and reducing device capable of enlarging and reducing the formed angle formed between the first
member and the second member, wherein
the enlarging and reducing device includes a power source, a power transmission unit configured to transmit
power of the power source, and a control unit configured to control the power source,
the joint device is provided to transition between a weighted state where a weight applied from an outside is received and a non-weighted state where no weight is received, and
the control unit,

(A) when the joint device is in the weighted state,
controls the power source based on a torque target value which is a target value of a torque related to a torque for the enlarging and reducing device to enlarge or reduce the formed angle, and
(B) when the joint device is in the non-weighted state,
controls the power source based on a position target value which is a target value of a position related to the formed angle enlarged or reduced by the enlarging and reducing device.

2. The joint device according to claim 1, wherein the torque target value is determined based on the weight applied.

3. The joint device according to 1, wherein the torque target value is determined based on a weight of a wearer.

4. The joint device according to any one of claims 1 to 3, wherein the torque target value is determined based on the formed angle or based on a supplementary angle of the formed angle.

5. The joint device according to any one of claims 1 to 4, wherein

the joint device is attached to a first portion of a wearer among the first portion and a second portion that pivots relative to the first portion,
the second member is provided to be attached to the first portion such that the first member is situated at a distal end side of the wearer than the second member, and
the torque target value is determined based on a length between the coupling portion and another coupling portion of the wearer, the another coupling portion coupling the first portion and the second portion such that another formed angle formed between the first portion and the second portion is variable.

6. The joint device according to any one of claims 1 to 5, wherein
the control unit,

(A) when the joint device is in the weighted state,
controls the power source to generate power in a direction such that the joint device in a state where the first member and the second member are bent becomes a state where the first member and the second member are stretched.

7. The joint device according to any one of claims 1 to 6, wherein the position target value is determined based on a first formed angle between the second member and a reference line passing through a pivoting axis of a first portion of a wearer and a second portion that pivots relative to the first portion.

8. The joint device according to any one of claims 1 to 7, wherein the position target value is determined based on a second formed angle between the first member and another reference line passing through another pivoting axis of the first member and the second member.

9. The joint device according to claim 8, wherein the second formed angle is determined such that the first member is positioned on a same side as the second member with respect to the another reference line.

10. The joint device according to claim 8 which is dependent on claim 7, wherein

when a period from a transition from the weighted state to the non-weighted state to a transition to the weighted state again is defined as a non-weighted period, and
a latter half of the non-weighted period is defined as a latter non-weighted period,
the position target value is determined based on the first formed angle or the second formed angle when the joint device is in the latter non-weighted period.

11. The joint device according to any one of claims 1 to 10, wherein the position target value is determined based on a third formed angle between the first member and another reference line passing through another pivoting axis of the first member and the second member.

12. The joint device according to claim 11, wherein the position target value is determined based on a difference between the third formed angle and a first formed angle between the second member and a reference line passing through a pivoting axis of a first portion of a wearer and a second portion that pivots relative to the first portion.

13. The joint device according to claim 11 or 12, wherein

when a period from a transition from the weighted state to the non-weighted state to a transition to the weighted state again is defined as a non-weighted period, and
an earlier half of the non-weighted period is defined as an earlier non-weighted period,
the position target value is determined based on the third formed angle when the joint device is in the earlier non-weighted period.

14. The joint device according to any one of claims 1 to 10, wherein

when a period from a transition from the weighted state to the non-weighted state to a transition to the weighted state again is defined as a non-weighted period, and
an earlier half of the non-weighted period is defined as an earlier non-weighted period, and a latter half is defined as a latter non-weighted period,
the control unit controls the power source based on the position target value determined by a determination method which is different between the earlier non-weighted period and the latter non-weighted period.

15. The joint device according to claim 14, wherein
the control unit switches to control for the latter non-weighted period, when a first formed angle between the second member and a reference line passing through a pivoting axis of a first portion of a wearer and a second portion that pivots relative to the first portion falls within a first predetermined range during the earlier non-weighted period.

16. The joint device according to claim 14 or 15, wherein

when a period from a transition from the non-weighted state to the weighted state to a transition to the non-weighted state again is defined as a weighted period,
the control unit switches to control for the weighted period when the applied weight falls within a second predetermined range during the latter non-weighted period.

17. The joint device according to any one of claims 1 to 16, wherein

when a period from a transition from the weighted state to the non-weighted state to a transition to the weighted state again is defined as a non-weighted period, and
a period from a transition from the non-weighted state to the weighted state to a transition to the non-weighted state again is defined as a weighted period,
the control unit switches to control for the non-weighted period when the applied weight falls within a third predetermined range during the weighted period.

18. The joint device according to claim 17, wherein
the control unit switches to control for the non-weighted period, when the applied weight falls within the third predetermined range after a first formed angle between the second member and a reference line passing through a pivoting axis of a first portion of a wearer and a second portion that pivots relative to the first portion falls within a fourth predetermined range during the weighted period.

19. The joint device according to claim 17 or 18, wherein
the control unit switches to control for the weighted period when the applied weight falls within a fifth predetermined range during the non-weighted period.

20. The joint device according to any one of claims 1 to 19, wherein

the enlarging and reducing device further includes a connection and disconnection mechanism disposed on a power transmission path of the power transmission unit and configured to switch between connection and disconnection of power in the power transmission path,
the control unit further controls the connection and disconnection mechanism, and

the control unit,
(B) when the joint device is in the non-weighted state,

> controls the connection and disconnection mechanism to a disconnection state where the power transmission path is disconnected, and
> controls the power source such that the power source does not generate power or is stopped, instead of controlling the power source based on the position target value.

21. The joint device according to claim 20, wherein
the control unit,

> (A) when the joint device is in the weighted state,

> > controls the connection and disconnection mechanism to a connection state where the power transmission path is connected, and
> > controls the power source such that the power source does not generate power or is stopped, instead of controlling the power source based on the torque target value.

22. The joint device according to claim 20 or 21, wherein the power source is a permanent-magnet-type electric motor.

23. The joint device according to any one of claims 20 to 22, wherein the control unit is configured to be capable of adjusting a braking force for stopping the power source.

24. The joint device according to claim 23, wherein the control unit causes to generate the braking force set according to a weight of a wearer.

25. The joint device according to any one of claims 1 to 24, wherein

> the power transmission unit has:

> > a first power transmission path for transmitting the power at a first transmission ratio; and
> > a second power transmission path for transmitting the power at a second transmission ratio different from the first transmission ratio,

> the enlarging and reducing device further includes:

> > a first connection and disconnection mechanism disposed on the first power transmission path and configured to switch between connection and disconnection of power in the first power transmission path; and
> > a second connection and disconnection mechanism disposed on the second power transmission path and configured to switch between connection and disconnection of power in the second power transmission path, and

> the control unit further controls the first connection and disconnection mechanism and the second connection and disconnection mechanism.

26. The joint device according to claim 25, wherein

> when the first transmission ratio is defined as a ratio of a post-transmission rotation speed with respect to a pre-transmission rotation speed which is a rotation speed on a power source side in the first power transmission path relative to a first transmission unit, and
> the second transmission ratio is defined as a ratio of a post-transmission rotation speed with respect to a pre-transmission rotation speed which is a rotation speed on the power source side in the second power transmission path relative to a second transmission unit,
> the first transmission ratio is configured to be smaller than the second transmission ratio.

27. The joint device according to claim 25 or 26, wherein at least one of the first transmission ratio and the second transmission ratio is determined based on a weight of a wearer.

28. The joint device according to any one of claims 25 to 27, wherein a ratio between the first transmission ratio and the second transmission ratio is set to 1.5 to 3.0.

29. The joint device according to any one of claims 1 to 28, wherein the joint device is a prosthetic limb device which is attached to a wearer such that the first member is situated at a distal end side of the wearer than the second member, and the coupling portion is provided to function as a joint of the wearer.

30. The joint device according to claim 29, wherein
the prosthetic limb device is a prosthetic leg device to be attached to a leg of the wearer.

31. The joint device according to claim 30, wherein
the prosthetic leg device is provided such that the second member is attached to a thigh of the leg, and the coupling portion is provided to function as a knee joint between the thigh and a lower leg.

32. A joint device comprising:

a first member;
a second member;
a coupling portion configured to couple the first member and the second member such that a formed angle formed between the first member and the second member is variable; and
an enlarging and reducing device capable of enlarging and reducing the formed angle formed between the first member and the second member, wherein
the enlarging and reducing device includes a power source, a power transmission unit configured to transmit power of the power source, a connection and disconnection mechanism disposed on a power transmission path of the power transmission unit and configured to switch between connection and disconnection of power in the power transmission path, and a control unit configured to control the power source and the connection and disconnection mechanism,
the joint device is provided to transition between a weighted state where a weight applied from an outside is received and a non-weighted state where no weight is received, and
the control unit,

(A) when the joint device is in the weighted state,

controls the connection and disconnection mechanism to a connection state where the power transmission path is connected, and
controls the power source such that the power source does not generate power or is stopped, and

(B) when the joint device is in the non-weighted state,

controls the connection and disconnection mechanism to a disconnection state where the power transmission path is disconnected, and
controls the power source such that the power source does not generate power or is stopped.

33. The joint device according to claim 32, wherein

the joint device is a prosthetic limb device which is attached to a wearer such that the first member is situated at a distal end side of the wearer than the second member, and the coupling portion is provided to function as a joint of the wearer,
the prosthetic limb device is a prosthetic leg device to be attached to a leg of the wearer, and
the control unit,

(A) when the joint device is in the weighted state,

(a) in a case of walking to advance on a flat ground, or in a case of walking to descend stairs,

controls the connection and disconnection mechanism to the connection state where the power transmission path is connected, and
controls the power source such that the power source does not generate power or is stopped, and

(b) in a case of walking to ascend stairs,

controls the connection and disconnection mechanism to the connection state where the power transmission path is connected, and
controls the power source based on a torque target value which is a target value of a torque related to a torque for the enlarging and reducing device to enlarge or reduce the formed angle.

34. The joint device according to claim 32 or 33, wherein

the joint device is a prosthetic limb device which is attached to a wearer such that the first member is situated at a distal end side of the wearer than the second member, and the coupling portion is provided to function as a joint of the wearer,
the prosthetic limb device is a prosthetic leg device to be attached to a leg of the wearer, and
the control unit,
(B) when the joint device is in the non-weighted state,

(a) in a case of walking to advance on a flat ground, or in a case of walking to descend stairs,

controls the connection and disconnection mechanism to the disconnection state where the power transmission path is disconnected, and
controls the power source such that the power source does not generate power or is stopped, and

(b) in a case of walking to ascend stairs,

controls the connection and disconnection mechanism to the connection state where the power transmission path is connected, and
controls the power source based on a position target value which is a target value of a position related to the formed angle enlarged or reduced by the enlarging and reducing device.

35. A joint device control method for controlling a joint device including:

a first member;
a second member;
a coupling portion configured to couple the first member and the second member such that a formed angle formed between the first member and the second member is variable; and
an enlarging and reducing device capable of enlarging and reducing the formed angle formed between the first member and the second member, wherein
the enlarging and reducing device includes a power source, and a power transmission unit configured to transmit power of the power source,
the joint device is provided to transition between a weighted state where a weight applied from an outside is received and a non-weighted state where no weight is received, and
the control method comprises the steps of:

(A) when the joint device is in the weighted state,
controlling the power source based on a torque target value which is a target value of a torque related to a torque for the enlarging and reducing device to enlarge or reduce the formed angle; and
(B) when the joint device is in the non-weighted state,
controlling the power source based on a position target value which is a target value of a position related to the formed angle enlarged or reduced by the enlarging and reducing device.

36. A joint device control program for controlling a joint device including:

a first member;
a second member;
a coupling portion configured to couple the first member and the second member such that a formed angle formed between the first member and the second member is variable; and
an enlarging and reducing device capable of enlarging and reducing the formed angle formed between the first member and the second member, wherein

the enlarging and reducing device includes a power source, and a power transmission unit configured to transmit power of the power source,

the joint device is provided to transition between a weighted state where a weight applied from an outside is received and a non-weighted state where no weight is received, and

the control program causes a computer to perform the steps of:

(A) when the joint device is in the weighted state,
controlling the power source based on a torque target value which is a target value of a torque related to a torque for the enlarging and reducing device to enlarge or reduce the formed angle; and
(B) when the joint device is in the non-weighted state,
controlling the power source based on a position target value which is a target value of a position related to the formed angle enlarged or reduced by the enlarging and reducing device.

**37.** A computer-readable storage medium storing the control program according to claim 36.

# FIG. 1

# FIG. 2

# FIG. 3

# FIG. 4

# FIG. 5

## FIG. 6

# FIG. 7

# FIG. 8

*FIG. 9*

# FIG. 10

（A）

（B）

FIG. 11

（A）

（B）

# FIG. 12

## （A）

## （B）

EP 4 585 190 A1

# FIG. 13

(A)

(B)

# FIG. 14

（A）

（B）

# FIG. 15

# FIG. 16

272 LOAD SENSOR

273 IMU

270

CONTROL UNIT — 10

PHASE DETERMINATION UNIT — 11

MODE ACQUISITION UNIT — 12

MOTOR CONTROL UNIT — 13

140 EXPANSION DEVICE

240 OPERATION MECHANISM

M MOTOR

T TRANSMISSION

SP SPINDLE UNIT

130 KNEE JOINT MECHANISM

270 KNEE ANGLE SENSOR

271

# FIG. 17

<STAIR ASCENDING OPERATION>

(A)    (B)    (C)    (D)    (E)    (F)    (G)    (H)

EP 4 585 190 A1

*FIG. 18*

<FLAT GROUND WALKING OPERATION>

(A)  (B)  (C)  (D)  (E)  (F)  (G)  (H)

123  130  1

# FIG. 19

(A) KNEE ANGLE

0deg          +deg

(B) THIGH ANGLE

+deg          0deg          −deg

(C) LOWER LEG ANGLE

+deg          0deg          −deg

## FIG. 20

<STAIR ASCENDING MODE>

| | STANCE PHASE | TRANSITION PHASE<br>{ STANCE ↓ INITIAL SWING } | INITIAL SWING PHASE | TRANSITION PHASE<br>{ INITIAL SWING ↓ TERMINAL SWING } | TERMINAL SWING PHASE | TRANSITION PHASE<br>{ TERMINAL SWING ↓ STANCE } |
|---|---|---|---|---|---|---|
| MOTOR M | DRIVEN IN STRETCHING DIRECTION (TORQUE CONTROL) | NON-DRIVEN | DRIVEN IN BENDING DIRECTION (POSITION CONTROL) | NON-DRIVEN | DRIVEN IN STRETCHING DIRECTION (POSITION CONTROL) | NON-DRIVEN |
| OPERATION MECHANISM 240 | HIGH-TORQUE-SIDE CONNECTION STATE | HIGH-TORQUE-SIDE CONNECTION STATE ↓ HIGH-SPEED-ROTATION-SIDE CONNECTION STATE | HIGH-SPEED-ROTATION-SIDE CONNECTION STATE | | | HIGH-SPEED-ROTATION-SIDE CONNECTION STATE ↓ HIGH-TORQUE-SIDE CONNECTION STATE |

<FLAT GROUND AND STAIR DESCENDING MODE>

| | STANCE PHASE | TRANSITION PHASE<br>{ STANCE ↓ SWING } | SWING PHASE | TRANSITION PHASE<br>{ SWING ↓ STANCE } |
|---|---|---|---|---|
| MOTOR M | NON-DRIVEN | | | |
| OPERATION MECHANISM 240 | HIGH-SPEED-ROTATION-SIDE CONNECTION STATE | HIGH-SPEED-ROTATION-SIDE CONNECTION STATE ↓ CUTOFF STATE | CUTOFF STATE | CUTOFF STATE ↓ HIGH-SPEED-ROTATION-SIDE CONNECTION STATE |

EP 4 585 190 A1

# FIG. 21

<STANCE PHASE>

# FIG. 22

<INITIAL SWING PHASE_METHOD 1>

EP 4 585 190 A1

# FIG. 23

<INITIAL SWING PHASE_METHOD 2>

(A)　　　　　　(B)　　　　　　(C)　　　　　　(D)

EP 4 585 190 A1

# FIG. 24

<TERMINAL SWING PHASE>

FIG. 25

EP 4 585 190 A1

## FIG. 26

## FIG. 27

| WALKING MODE | PHASE | SWITCH SW1 | TRANSISTORS Tr1 to Tr3 | MOTOR M |
|---|---|---|---|---|
| STAIR ASCENDING MODE | STANCE PHASE | ON | All OFF | DRIVEN IN STRETCHING DIRECTION (TORQUE CONTROL) |
| | SWING PHASE | ON | All OFF | DRIVEN IN BENDING OR STRETCHING DIRECTION (POSITION CONTROL) |
| FLAT GROUND AND STAIR DESCENDING MODE | STANCE PHASE | OFF | THE NUMBER OF TRANSISTORS IN ON STATE VARIES ACCORDING TO BODY WEIGHT | NON-DRIVEN |
| | SWING PHASE | OFF | All OFF | NON-DRIVEN |

EP 4 585 190 A1

# FIG. 28

| BODY WEIGHT | TRANSISTOR Tr1 | TRANSISTOR Tr2 | TRANSISTOR Tr3z |
|---|---|---|---|
| LIGHT ↕ HEAVY | OFF | OFF | OFF |
| | ON | OFF | OFF |
| | ON | ON | OFF |
| | ON | ON | ON |

## INTERNATIONAL SEARCH REPORT

| International application No. |
| --- |
| **PCT/JP2023/032583** |

**A.    CLASSIFICATION OF SUBJECT MATTER**

*A61F 2/64*(2006.01)i; *A61F 2/70*(2006.01)i

FI:    A61F2/64; A61F2/70

According to International Patent Classification (IPC) or to both national classification and IPC

**B.    FIELDS SEARCHED**

Minimum documentation searched (classification system followed by classification symbols)

A61F2/64; A61F2/70

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| P, X | WO 2022/186081 A1 (HONDA MOTOR CO., LTD.) 09 September 2022 (2022-09-09) paragraphs [0012]-[0017], [0023]-[0029], [0072]-[0115], fig. 1-13, 15-17 | 1-7, 14-17, 19, 25-26, 29-31, 35-37 |
| L | | 1-7, 14-17, 19, 25-26, 29-31, 35-37 |
| P, X | WO 2023/113001 A1 (HONDA MOTOR CO., LTD.) 22 June 2023 (2023-06-22) paragraphs [0010]-[0015], [0063]-[0069], fig. 1-5, 17, 18 | 32-33 |
| L | | 32-33 |
| X | WO 2018/092325 A1 (CYBERDYNE INC.) 24 May 2018 (2018-05-24) paragraphs [0026]-[0038], [0106]-[0110], fig. 1-10, 13 | 1, 4, 6-7, 17, 19, 29-31, 35-37 |
| A | | 2-3, 5, 8-16, 18, 20-28, 32-34 |
| X | US 2022/0202596 A1 (KOREA LABOR WELFARE CORPORATION CO., LTD.) 30 June 2022 (2022-06-30) paragraphs [0052]-[0062], [0117]-[0123], fig. 1-10 | 32 |
| A | | 1-31, 33-37 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **15 November 2023** | **28 November 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2023/032583**

## C.     DOCUMENTS CONSIDERED TO BE RELEVANT

| Category* | Citation of document, with indication, where appropriate, of the relevant passages | Relevant to claim No. |
|---|---|---|
| A | US 2015/0127118 A1 (IWALK, INC.) 07 May 2015 (2015-05-07)<br>entire text, all drawings | 1-37 |
| A | WO 2020/203762 A1 (HONDA MOTOR CO., LTD.) 08 October 2020 (2020-10-08)<br>entire text, all drawings | 1-37 |
| A | US 2016/0158029 A1 (REHABILITATION INSTITUTE OF CHICAGO) 09 June 2016 (2016-06-09)<br>entire text, all drawings | 1-37 |

Form PCT/ISA/210 (second sheet) (January 2015)

## INTERNATIONAL SEARCH REPORT
### Information on patent family members

International application No.

**PCT/JP2023/032583**

| Patent document cited in search report | | | Publication date (day/month/year) | Patent family member(s) | | | Publication date (day/month/year) |
|---|---|---|---|---|---|---|---|
| WO | 2022/186081 | A1 | 09 September 2022 | (Family: none) | | | |
| WO | 2023/113001 | A1 | 22 June 2023 | (Family: none) | | | |
| WO | 2018/092325 | A1 | 24 May 2018 | US paragraphs [0039]-[0051], [0092]-[0128], fig. 1-10, 13 EP | 2019/0328553 3542762 | A1 A1 | |
| US | 2022/0202596 | A1 | 30 June 2022 | WO EP KR | 2021/002536 3995113 10-2021-0003568 | A1 A1 A | |
| US | 2015/0127118 | A1 | 07 May 2015 | WO CA | 2013/188510 2876187 | A2 A1 | |
| WO | 2020/203762 | A1 | 08 October 2020 | EP entire text, all drawings CN | 3950233 113710433 | A1 A | |
| US | 2016/0158029 | A1 | 09 June 2016 | WO CA | 2016/094413 2969884 | A1 A1 | |

Form PCT/ISA/210 (patent family annex) (January 2015)

**EP 4 585 190 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021251500 A
- JP 2022142463 A